# EUROPÄISCHE PATENTSCHRIFT

(11) **EP 2 411 427 B1**
(45) Veröffentlichungstag und Bekanntmachung des Hinweises auf die Patenterteilung: **19.12.2012**
(21) Anmeldenummer: 10710802.9
(22) Anmeldetag: 20.03.2010
(51) Int. Cl.: C08F 220/26, C08F 230/02

(54) **POLYMERE AUF BASIS VON ACRYL-, METHACRYL- ODER ETHACRYLAMIDOALKYLSULFONSÄURE ODER -SALZEN UND CARBOXYALKYLACRYLAT, -METHACRYLAT ODER -ETHACRYLAT ODER OLIGOMEREN DIESER CARBOXY-VERBINDUNGEN**
POLYMERS BASED ON ACRYLIC, METHACRYLIC OR ETHACRYLIC AMIDOALKYL SULFONIC ACID OR SALTS AND CARBOXYALKYL ACRYLATE, METHACRYLATE OR ETHACRYLATE OR OLIGOMERS OF SAID CARBOXY COMPOUNDS
POLYMÈRES À BASE D'ACIDE ACRYL-, MÉTHACRYL- OU ÉTHACRYLAMIDOALKYLSULFONIQUE OU DE SELS DE CES DERNIERS, ET D'ACRYLATE, MÉTHACRYLATE OU MÉTHACRYLATE DE CARBOXYALKYLE, OU D'OLIGOMÈRES DE CES COMPOSÉS CARBOXYLÉS

(30) Priorität: 25.03.2009 DE 102009014877
(43) Veröffentlichungstag der Anmeldung: 01.02.2012
(73) Patentinhaber: Clariant Finance (BVI) Limited, Road Town, Tortola (VG)
(72) Erfinder: KLUG, Peter, 63762 Großostheim (DE); FISCHER, Dirk, 55270 Klein-Winternheim (DE); LINDNER, Thomas, 68199 Mannheim (DE); KUNZE, Matthias, 60528 Frankfurt (DE); MOELLER, Wiebke, 65812 Bad Soden (DE); MILDNER, Carina, 65931 Frankfurt am Main (DE); LO VASCO, Sebastiano, 63695 Glauburg (DE)
(74) Vertreter: Paczkowski, Marcus
(86) Internationale Anmeldenummer: PCT/EP2010/001755
(87) Internationale Veröffentlichungsnummer: WO 2010/108634

(56) Entgegenhaltungen:
- WO-A1-90/12822
- US-A- 5 185 395

## Beschreibung

Die vorliegende Erfindung betrifft vernetzte wasserlösliche oder wasserquellbare Polymere auf Basis von Acryl-, Methacryl- oder Ethacrylamidoalkylsulfonsäure oder -salzen und Carboxyalkylacrylat, -methacrylat oder -ethacrylat oder Oligomeren dieser Carboxy-Verbindungen, ein Verfahren zur Herstellung dieser Polymere, die Verwendung dieser Polymere als Verdicker, Konsistenzgeber oder Fließgrenzenbildner, vorzugsweise als Verdicker oder Fließgrenzenbildner in wässrigen Tensidsystemen oder als Fließgrenzenbildner für anorganische Partikel, organische Partikel, Öltröpfchen, oder Gasblasen in Tensidsystemen, besonders bevorzugt in kosmetischen, dermatologischen oder pharmazeutischen Zusammensetzungen, sowie kosmetische, dermatologische oder pharmazeutische Zusammensetzungen enthaltend ein oder mehrere dieser Polymere.

Polymere auf Basis von 2-Acrylamido-2-methyl-propan-sulfonsäure oder ihrer Salze sind bereits bekannt.

Beispielsweise sind vernetzte Homopolymere der 2-Acrylamido-2-methyl-propan-sulfonsäure oder ihrer Salze (Hostacerin^{®} AMPS, Clariant) sowie vernetzte Copolymere mit weiteren Monomeren wie z. B. Vinylpyrrolidon (Aristoflex^{®} AVC, Clariant) käuflich erwerbbar und in EP 0 816 403 und EP 1 116 733 offenbart. Nachteilig an diesen Polymeren ist jedoch, dass sie in Tensidsystemen keine klaren Lösungen und keine ausreichenden Fließgrenzen ausbilden.

Des Weiteren sind auch hydrophob modifizierte Polymere auf Basis von 2-Acrylamido-2-methyl-propan-sulfonsäure oder ihrer Salze käuflich erwerbbar (z. B. Aristoflex^{®} HMB, Clariant) und z. B. in EP 1 069 142 beschrieben. Nachteilig an diesen Polymeren ist jedoch, dass sie in Tensidsystemen nur in speziellen Fällen eine ausreichende Fließgrenze und klare Lösungen bilden.

Zur Generierung der beschriebenen Effekte, d. h. zur Ausbildung von klaren Lösungen und von Fließgrenzen insbesondere in Tensidsystemen, werden im Markt quervernetzte Polyacrylsäuren und deren Copolymere (z. B. Carbopol^{®} Aqua SF1 von Lubrizol) verwendet. Diese haben jedoch wiederum den Nachteil, dass sie unterhalb eines pH-Wertes von ca. 6 keine Verdickung, keine Fließgrenzen und auch keine klaren Formulierungen erzeugen.

Des Weiteren sind die im Markt befindlichen Copolymere auf Basis von 2-Acrylamido-2-methyl-propan-sulfonsäure oder deren Salzen und weiteren Monomeren ausgewählt aus Acrylsäure und Methacrylsäure nur in Form von ölhaltigen inversen Emulsionen (z. B. Simulgel^{®} von Seppic) erhältlich und nicht zum Einsatz in Tensidsystemen geeignet, da der hohe Ölgehalt zu Trübungen in den Tensidsystemen führt.

Aufgabe der vorliegenden Erfindung war es daher, Substanzen bereitzustellen, mit deren Hilfe die Nachteile der Substanzen des Standes der Technik verringert oder sogar vermieden werden können und die insbesondere in vorteilhafter Weise zur Herstellung von tensidhaltigen Zusammensetzungen, beispielsweise von kosmetischen, dermatologischen oder pharmazeutischen tensidhaltigen Zusammensetzungen, verwendet werden können, und dadurch klare tensidhaltige Zusammensetzungen erhalten werden können, die eine Fließgrenze aufweisen, und in denen insbesondere anorganische und/oder organische Partikel und/oder Öltröpfchen sowie Gasblasen stabilisiert werden können, und die in einem pH-Bereich von 2 bis 11, insbesondere in sauren pH-Bereichen von 2 bis 6, sehr gute rheologische Eigenschaften zeigen, zugleich hautfreundlich sind und zusätzlich im Vergleich zu Polyacrylaten eine vorteilhaftere Hautsensorik aufweisen.

Überraschenderweise wurde nun gefunden, dass diese Aufgabe gelöst wird durch bestimmte vernetzte wasserlösliche oder wasserquellbare Polymere auf Basis von Acryl-, Methacryl- oder Ethacrylamidoalkylsulfonsäuren oder deren Salzen und Carboxyalkylacrylat, -methacrylat oder -ethacrylat und/oder Oligomeren dieser Carboxy-Verbindungen.

Gegenstand der Erfindung sind daher wasserlösliche oder wasserquellbare Polymere enthaltend
a) 20,0 bis 98,99 mol-%, vorzugsweise 20,0 bis 98,98 mol-%, einer oder mehrerer voneinander unabhängiger wiederkehrender Struktureinheiten der Formel (1) 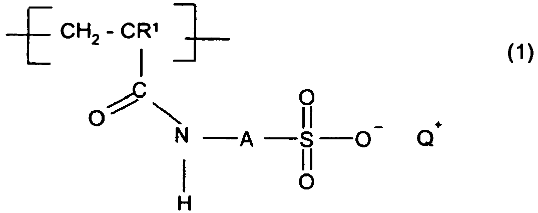 worin
   - R¹: Wasserstoff, Methyl oder Ethyl bedeutet,
   - A: lineares oder verzweigtes C₁-C₁₂-Alkylen, vorzugsweise C₁-C₈-Alkylen, bedeutet, und
   - Q⁺: für H⁺, NH₄⁺, organische Ammoniumionen [HNR⁵R⁶R⁷]⁺, wobei R⁵, R⁶ und R⁷ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine C₆-C₂₂-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste R⁵, R⁶ und R⁷ nicht Wasserstoff ist, Li⁺, Na⁺, K⁺, ½ Ca⁺⁺, ½ Mg⁺⁺, ½ Zn⁺⁺ oder ⅓ Al⁺⁺⁺ oder für Mischungen aus diesen Ionen steht,
   und
b) 1,0 bis 79,99 mol-%, vorzugsweise 1,0 bis 79,98 mol-%, einer oder mehrerer voneinander unabhängiger wiederkehrender Struktureinheiten der Formel (2) 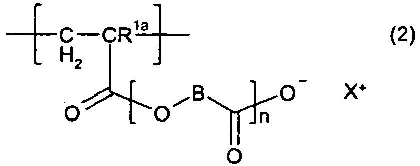 worin
   - R^{1a}: Wasserstoff, Methyl oder Ethyl bedeutet,
   - X⁺: für H⁺, NH₄⁺, organische Ammoniumionen [HNR⁵R⁶R⁷]⁺, wobei R⁵, R⁶ und R⁷ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine C₆-C₂₂-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste R⁵, R⁶ und R⁷ nicht Wasserstoff ist, Li⁺, Na⁺, K⁺, ½ Ca⁺⁺, ½ Mg⁺⁺, ½ Zn⁺⁺ oder ⅓ Al⁺⁺⁺ oder für Mischungen aus diesen Ionen steht,
   - B: eine lineare oder verzweigte Alkylengruppe mit 1 bis 6 Kohlenstoffatomen ist, und
   - n: eine ganze Zahl von 1 bis 10 ist,
   und
c) 0,01 bis 8,0 mol-%, vorzugsweise 0,01 bis 5,0 mol-%, besonders bevorzugt 0,01 bis 2,0 mol-% und insbesondere bevorzugt 0,25 bis 1,5 mol-% an einer oder mehreren voneinander unabhängigen wiederkehrenden vernetzenden Struktureinheiten, die aus einem oder mehreren Monomeren mit mindestens zwei olefinischen Doppelbindungen hervorgegangen sind.

In einem erfindunsgemäßen Polymer können jeweils verschiedene Struktureinheiten der Formel (1) und/oder der Formel (2) enthalten sein. Ein erfindungsgemäßes Polymer kann beispielsweise mehrere Struktureinheiten der Formel (1) enthalten, die sich durch unterschiedliche Gegenionen Q⁺ voneinander unterscheiden. Ein erfindungsgemäßes Polymer kann beispielsweise auch mehrere Struktureinheiten der Formel (2) enthalten, die sich durch unterschiedliche Gegenionen X⁺ voneinander unterscheiden.

Die erfindungsgemäßen Polymere sind u. a. hervorragend geeignet als Verdicker und/oder Fließgrenzenbildner von wässrigen Systemen und von kosmetischen, dermatologischen oder pharmazeutischen Zusammensetzungen, und insbesondere als Verdicker und/oder Fließgrenzenbildner von klaren wässrigen tensidhaltigen Systemen, welche anorganische und/oder organische Partikel und/oder Öltröpfchen und/oder Gasblasen enthalten. Vorteilhafterweise zeigen sie auch über einen weiten pH-Bereich, d. h. auch bei stark sauren pH-Werten, gute Verdickungs- und/oder Stabilisierungseigenschaften.

Polymere auf Basis von 2-Carboxyethylacrylat oder dessen Oligomeren sind ebenfalls bereits bekannt.

In US 2008/0014154 A1 werden Copolymere im Wesentlichen bestehend aus speziellen Struktureinheiten enthaltend Polyethylenglykol-Gruppierungen und Struktureinheiten abgeleitet von im Wesentlichen kationischen Monomeren beschrieben. Diese im Wesentlichen kationischen Monomere können Mischungen aus einem oder mehreren speziellen kationischen Monomeren und einem oder mehreren speziellen anionischen Monomeren darstellen. Die Copolymere sind nicht vernetzt und können in kosmetischen oder pharmazeutischen Zusammensetzungen eingesetzt werden.

In EP 0 036 294 A2 werden Oligomere der Formel CH₂ = CH-COO-(CH₂CH₂COO)ₙH, wobei n mittlere Werte n von größer 1 bis 10 besitzt, offenbart und ihre Verwendung zur Herstellung von Homo- oder Copolymeren. Die Polymere können in Beschichtungen und/oder Imprägnierungs-Zusammensetzungen verwendet werden.

In EP 0 699 726 A2 werden wassedösliche Haftklebemassen beschrieben, die ein wasserlösliches Copolymerisat enthalten, das u. a. aus 65 - 95 Gew.-% einer Vinylcarbonsäure synthetisiert ist.

In EP 0 574 202 A1 wird ein Verfahren zur Herstellung eines hydrophilen Harzes beschrieben, wobei u. a. ein hydrophiles ungesättigtes Monomer mit 0,001 bis 50 mol-%, bezogen auf die Menge der Monomere, an speziellen Vernetzern copolymerisiert werden und das Produkt der Copolymerisation hitzebehandelt wird. Die Vernetzer besitzen Struktureinheiten CH₂ = CHCOO-(CH₂CH₂COO)ₙ-, wobei n mindestens 1 ist. Das hydrophile Harz kann z. B. verwendet werden, um absorbierende Harze zur Aufnahme von Flüssigkeiten herzustellen, indem das hydrophile Harz mit einem weiteren Vernetzer oberflächenbehandelt wird.

In US 2007/0166269 A1 werden kosmetische Zusammensetzungen beschrieben, die spezielle Copolymeren enthalten und in der Form von Wasser-in-Öl-Emulsionen oder multiplen Emulsionen vorliegen. Die Copolymere enthalten u. a. mindestens eine Monomereinheit, die von einem wenigstens teilweise neutralisierten ionischen hydrophilen Monomer abgeleitet sind.

In der einen oder den mehreren Struktureinheiten der Formel (1) der erfindungsgemäßen Polymere ist R¹ vorzugsweise Wasserstoff oder Methyl und besonders bevorzugt Wasserstoff.

In der einen oder den mehreren Struktureinheiten der Formel (1) der erfindungsgemäßen Polymere ist A vorzugsweise eine Struktureinheit der Formel -CH₂-C(CH₃)₂-.

Besonders bevorzugt ist die eine oder sind die mehreren Struktureinheiten der Formel (1) der erfindungsgemäßen Polymere abgeleitet von der 2-Acrylamido-2-methyl-propan-sulfonsäure.

Vorzugsweise ist der Neutralisationsgrad der einen oder der mehreren Struktureinheiten der Formel (1) der erfindungsgemäßen Polymere von 50,0 bis 100 mol-%, besonders bevorzugt von 80,0 bis 100 mol-%, insbesondere bevorzugt von 90,0 bis 100 mol-% und außerordentlich bevorzugt von 95,0 bis 100 mol-%.

In der einen oder den mehreren Struktureinheiten der Formel (1) der erfindungsgemäßen Polymere ist das von H⁺ verschiedene Gegenion Q⁺ vorzugsweise ausgewählt aus NH₄⁺, Alkali⁺, wobei unter Alkali⁺ wiederum Na⁺ bevorzugt ist, Erdalkali⁺⁺ und Mischungen aus diesen Ionen. Besonders bevorzugt ist das von H⁺ verschiedene Gegenion Q⁺ NH₄⁺.

In der einen oder den mehreren Struktureinheiten der Formel (2) der erfindungsgemäßen Polymere ist R^{1a} vorzugsweise Wasserstoff oder Methyl und besonders bevorzugt Wasserstoff.

In der einen oder den mehreren Struktureinheiten der Formeln (2) der erfindungsgemäßen Polymere ist B vorzugsweise eine Struktureinheit der Formel -CH₂-CH₂-.

In der einen oder den mehreren Struktureinheiten der Formel (2) der erfindungsgemäßen Polymere ist n vorzugsweise 1 bis 5 und besonders bevorzugt 1 bis 4.

Vorzugsweise ist der Anteil an der einen oder den mehreren Struktureinheiten der Formel (2), worin n eine ganze Zahl von 2 oder mehr ist, innerhalb der Komponente b) der erfindungsgemäßen Polymere mindestens 10,0 mol-%, besonders bevorzugt mindestens 20,0 mol-%, insbesondere bevorzugt mindestens 25,0 mol-%, außerordentlich bevorzugt mindestens 30,0 mol-% und vorzugsweise höchstens 70,0 mol-%.

Besonders bevorzugt ist in der einen oder den mehreren Struktureinheiten der Formel (2) B die Gruppe -CH₂CH₂- und n eine ganze Zahl von 1 bis 5 und vorzugsweise von 1 bis 4.

In der einen oder den mehreren Struktureinheiten der Formel (2) der erfindungsgemäßen Polymere ist das Kation X⁺ vorzugsweise ausgewählt aus H⁺, NH₄⁺, Alkali⁺, wobei unter Alkali⁺ wiederum Na⁺ bevorzugt ist, Erdalkali⁺ und Mischungen aus diesen Ionen. Besonders bevorzugt ist das Gegenion X⁺ H⁺ und/oder NH₄⁺.

Besonders bevorzugt ist das von H⁺ verschiedene Gegenion Q⁺ in der einen oder den mehreren Struktureinheiten der Formel (1) ausgewählt aus NH₄⁺, Alkali⁺, wobei unter Alkali⁺ wiederum Na⁺ bevorzugt ist, Erdalkali⁺⁺ und Mischungen aus diesen Ionen und besonders bevorzugt ist es NH₄⁺ und das Gegenion X⁺ in der einen oder den mehreren Struktureinheiten der Formel (2) ausgewählt aus H⁺, NH₄⁺, Alkali⁺, wobei unter Alkali⁺ wiederum Na⁺ bevorzugt ist, Erdalkali⁺⁺ und Mischungen aus diesen Ionen und besonders bevorzugt ist es H⁺ und/oder NH₄⁺.

In einer weiteren besonders bevorzugten Ausführungsform der Erfindung ist X⁺ in der einen oder den mehreren Struktureinheiten der Formel (2) H⁺.

In einer weiteren besonders bevorzugten Ausführungsform der Erfindung enthalten die erfindungsgemäßen Polymere mehrere Struktureinheiten der Formel (2), wobei in einigen Struktureinheiten der Formel (2) die Bedeutung der Gegenionen X⁺ H⁺ ist und in den anderen Struktureinheiten der Formel (2) die Bedeutung der Gegenionen X⁺ eine andere als H⁺ und vorzugsweise NH₄⁺ ist.

In einer bevorzugten Ausführungsform der Erfindung beträgt das molare Verhältnis der einen oder der mehreren Struktureinheiten der Formel (1) zu der einen oder den mehreren Struktureinheiten der Formel (2) von 20 : 80 bis 99 : 1, besonders bevorzugt von 50 : 50 bis 97 : 3, insbesondere bevorzugt von 75 : 25 bis 95 : 5 und außerordentlich bevorzugt von 85 : 15 bis 95 : 5.

Vorzugsweise enthalten die erfindungsgemäßen Polymere
a) 50,0 bis 96,99 mol-%, vorzugsweise 75,0 bis 94,99 mol-% und besonders bevorzugt 84,75 bis 94,75 mol-% an der einen oder den mehreren Struktureinheiten der Formel (1),
b) 3,0 bis 49,99 mol-%, vorzugsweise 5,0 bis 24,99 mol-% und besonders bevorzugt 5,0 bis 15,0 mol-% an der einen oder den mehreren Struktureinheiten der Formel (2), und
c) 0,01 bis 5,0 mol-%, vorzugsweise 0,01 bis 2,0 mol-% und besonders bevorzugt 0,25 bis 1,5 mol-% an der einen oder den mehreren vernetzenden Struktureinheiten der Komponente c).

In einer weiteren bevorzugten Ausführungsform der Erfindung ist die Menge der einen oder der mehreren Struktureinheiten der Formel (2) in den erfindungsgemäßen Polymeren kleiner oder gleich 50 Gew.-%, vorzugsweise kleiner oder gleich 45 Gew.-%, jeweils bezogen auf die Gesamtmasse der erfindungsgemäßen Polymere.

Die eine oder die mehreren vernetzenden Struktureinheiten der Komponente c) der erfindungsgemäßen Polymere leiten sich vorzugsweise ab von Acryl- oder Methacrylsäureestern, Acryl- oder Methacrylsäureamiden, Polyglykoldiacryl- oder -methacrylsäureestern, Polyglykoldiacryl- oder -methacrylsäureamiden, Dipropylenglykoldiacryl- oder -methacrylsäureestern, Dipropylenglykoldiacryl- oder -methacrylsäureamiden, ethoxylierten Glycerindiacrylaten oder -methacrylaten, ethoxylierten Glycerintriacrylaten oder -methacrylaten, propoxylierten Glycerindiacrylaten oder -methacrylaten, propoxylierten Glycerintriacrylaten oder -methacrylaten, oder anderen Acryl- oder Methacrylsäureestern, Acryl- oder Methacrylsäureamiden multifunktioneller Alkohole, Trimethylolpropantriacrylaten oder -trimethacrylaten, Allyl- oder Vinylethern multifunktioneller Alkohole, Methylen-bis-acrylamid oder Divinylbenzol.

Weiterhin bevorzugt leiten sich die eine oder die mehreren vernetzenden Struktureinheiten der Komponente c) der erfindungsgemäßen Polymere ab von Monomeren der allgemeinen Formel (9) 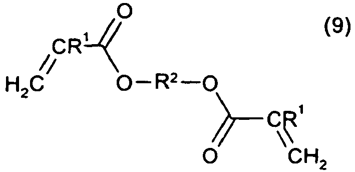 worin
- R¹: Wasserstoff, Methyl oder Ethyl bedeutet und
- R²: eine lineare oder verzweigte Alkylengruppe mit 1 bis 6 Kohlenstoffatomen oder eine lineare oder verzweigte, einfach oder mehrfach ungesättigte Alkenylengruppe mit 2 bis 6 Kohlenstoffatomen ist.

Weiterhin bevorzugt enthalten die erfindungsgemäßen Polymere ein oder mehrere voneinander unabhängige wiederkehrende vernetzende Struktureinheiten der Formel (3) 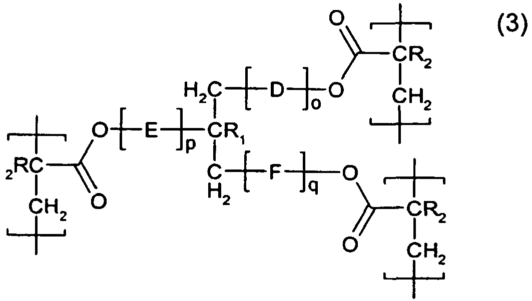 gemäß Komponente c), worin
- R¹ und R²: jeweilsunabhängig voneinander Wasserstoff, Methyl oder Ethyl bedeuten,
- D, E und F: jeweils unabhängig voneinander Methylenoxy (-CH₂-O-), Ethylenoxy (-CH₂-CH₂-O-), Popylenoxy (-CH(CH₃)-CH₂-O-), eine lineare oder verzweigte Alkylengruppe mit 1 bis 6 Kohlenstoffatomen, eine lineare oder verzweigte, einfach oder mehrfach ungesättigte Alkenylengruppe mit 2 bis 6 Kohlenstoffatomen, eine lineare Mono-Hydroxyalkylengruppe mit 2 bis 6 Kohlenstoffatomen oder eine lineare oder verzweigte Di-Hydroxyalkylengruppe mit 3 bis 6 Kohlenstoffatomen bedeuten, und
- o, p und q: jeweils unabhängig voneinander eine ganze Zahl von 1 bis 50 bedeuten.

Besonders bevorzugt als Vernetzer für die erfindungsgemäßen Polymere sind Glycerinpropoxylattriacrylat (GPTA), Trimethylolpropantriacrylat (TMPTA), Pentaerythritoldiacrylatmonostearat (PEAS), Hexandioldiacrylat (HDDA) und Hexandioldimethacrylat (HDDMA). Insbesondere bevorzugt ist Glycerinpropoxylattriacrylat (GPTA).

Besonders bevorzugte erfindungsgemäße Polymere enthalten
ab) 29,99 bis 98,99 mol-%, vorzugsweise 62,0 bis 98,99 mol-%, einer Mischung der wiederkehrenden Struktureinheiten der Formeln (1) und (2),
c) 0,01 bis 8,0 mol-% an der einen oder den mehreren vernetzenden Struktureinheiten der Komponente c), und
d) 0,01 bis 70,0 mol-%, vorzugsweise 1,0 bis 70,0 mol-%, besonders bevorzugt 1,0 bis 37,99 mol-%, einer oder mehrerer voneinander unabhängiger wiederkehrender nicht-vernetzender Struktureinheiten, die hervorgegangen sind aus einer oder mehreren Verbindungen der allgemeinen Formel (4) 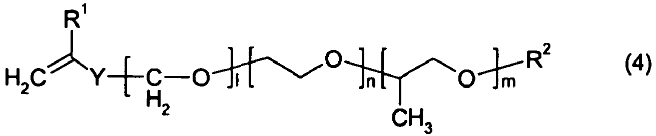 worin
   - R¹: Wasserstoff, Methyl oder Ethyl bedeutet,
   - R²: H, eine lineare oder verzweigte Alkylgruppe mit 1 bis 50 Kohlenstoffatomen, eine lineare oder verzweigte Mono-Hydroxyalkylgruppe mit 2 bis 6 Kohlenstoffatomen, eine lineare oder verzweigte Di-Hydroxyalkylgruppe mit 2 bis 6 Kohlenstoffatomen, -(CO-O-R⁷-)ₒR⁸ oder -(CO-NR⁵-R⁷-)ₚR⁸ bedeutet,
   - l, m, n, o und p: jeweils unabhängig voneinander eine ganze Zahl von 0 bis 300 bedeuten,
   - Y: eine chemische Bindung, O, NR³, S, PR³, CH₂, CH₂O, CH₂NR³, CH₂S, C(O), C(NR³), C(O)O, OC(O), C(O)NR³, NR³C(O), C(NR⁴)NR³, C(O)S, R⁶OC(O)O, R⁶OC(O)NR³, R⁶R³NC(O)NR⁴, R⁶NR³C(NR⁵)NR⁴, R⁶OC(O)S, R⁶P(O)O, R⁶OP(O)O, R⁶S(O), R⁶S(O)(O), R⁶S(O)O, R⁶S(O)(O)O, R⁶OS(O)O oder R⁶OS(O)(O)O, vorzugsweise eine chemische Bindung, O, CH₂, C(O)O, OC(O), C(O)NR³ oder NR³C(O) bedeutet,
   - R³, R⁴, R⁵ und R⁸: jeweils unabhängig voneinander Wasserstoff oder einen linearen oder verzweigten Alkylrest mit 1 bis 50 C-Atomen bedeuten,
   - R⁶: eine chemische Bindung oder CH₂ bedeutet und
   - R⁷: einen linearen oder verzweigten Alkylenrest mit 1 bis 50 C-Atomen bedeutet.

In der einen oder den mehreren Verbindungen der Formel (4) ist R¹ vorzugsweise Wasserstoff oder Methyl.

In der einen oder den mehreren Verbindungen der Formel (4) ist R² vorzugsweise H, eine lineare oder verzweigte Alkylgruppe mit 1 bis 50 Kohlenstoffatomen, eine lineare oder verzweigte Mono-Hydroxyalkylgruppe mit 2 bis 6 Kohlenstoffatomen oder eine lineare oder verzweigte Di-Hydroxyalkylgruppe mit 2 bis 6 Kohlenstoffatomen.

In der einen oder den mehreren Verbindungen der Formel (4) bedeutet l vorzugsweise 0.

In der einen oder den mehreren Verbindungen der Formel (4) bedeutet Y vorzugsweise eine chemische Bindung, O, CH₂, C(O)O, OC(O), C(O)NR³ oder NR³C(O).

Insbesondere bevorzugt ist die eine oder sind die mehreren Struktureinheiten der Komponente d) ausgewählt aus einer oder mehreren Struktureinheiten der Formel (5) 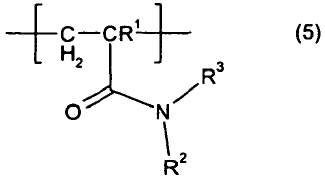 worin
- R¹: Wasserstoff, Methyl oder Ethyl bedeutet und
- R² und R³: jeweils unabhängig voneinander Wasserstoff, Methyl, Ethyl, n-Propyl oder iso-Propyl bedeuten, und mindestens einer der Reste R² und R³ nicht Wasserstoff ist, und
einer oder mehreren der Struktureinheiten der Formel (6) 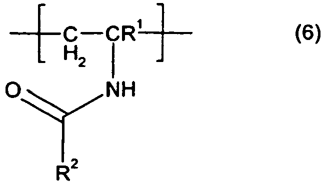 worin
- R¹: Wasserstoff, Methyl oder Ethyl bedeutet und
- R²: Wasserstoff, Methyl, Ethyl, n-Propyl oder iso-Propyl ist.

In einer bevorzugten Ausführungsform der Erfindung ist die eine oder sind die mehreren Struktureinheiten der Komponente d) ausgewählt aus einer oder mehreren Struktureinheiten der Formel (5).

In einer weiteren bevorzugten Ausführungsform der Erfindung ist die eine oder sind die mehreren Struktureinheiten der Komponente d) ausgewählt aus einer oder mehreren Struktureinheiten der Formel (6).

In einer weiteren bevorzugten Ausführungsform der Erfindung enthalten die erfindungsgemäßen Polymere Struktureinheiten abgeleitet von Acrylsäure. Sofern die erfindungsgemäßen Polymere derartige Struktureinheiten enthalten, sind sie vorzugsweise in einer Menge von 0,01 bis 10,0 mol-% in den erfindungsgemäßen Polymeren enthalten, bezogen auf die Gesamtmasse der Polymere.

In einer weiteren bevorzugten Ausführungsform der Erfindung enthalten die erfindungsgemäßen Polymere keine kationischen Struktureinheiten.

Die Verteilung der verschiedenen Struktureinheiten in den erfindungsgemäßen Polymeren kann statistisch, blockartig, alternierend oder gradientenartig sein.

Die erfindungsgemäßen Polymere besitzen bevorzugt ein Molekulargewicht von 10³ bis 10⁹ g/mol, besonders bevorzugt von 10⁴ bis 10⁷ g/mol und insbesondere bevorzugt von 10⁵ bis 5 - 10⁶ g/mol.

Die Herstellung der erfindungsgemäßen Polymere erfolgt mittels radikalischer Polymerisation in einem protischen Lösungsmittel, vorzugsweise in tert.-Butanol. Hierbei werden die entsprechenden Monomere z. B. in dem protischen Lösungsmittel gelöst oder dispergiert und die Polymerisation in an sich bekannter Weise, z. B. durch Zugabe einer radikalbildenden Verbindung, gestartet. Dabei können beispielsweise die vorgelegten Monomere "direkt" polymerisiert werden. Sie können aber auch vor der Polymerisation neutralisiert werden, indem beispielsweise saure Gruppen eingesetzter Monomere vor der Polymerisation mit Basen ungesetzt werden, wobei die Gegenionen Q⁺ der Struktureinheiten gemäß Formel (1) oder X⁺ der Struktureinheiten gemäß Formel (2) ausgebildet werden. Anstelle der Neutralisation der Monomere vor der Polymerisation können jedoch auch die Polymere nach der erfolgten Polymerisation mit den Basen neutralisiert werden.

Weiterer Gegenstand der vorliegenden Erfindung ist daher ein Verfahren zur Herstellung der erfindungsgemäßen Polymere, wobei Monomere, aus denen sich die Struktureinheiten der Komponenten a) bis c) und gegebenenfalls d) ableiten, sowie gegebenenfalls weitere Monomere, in einem protischen Lösungsmittel, vorzugsweise in tert.-Butanol, radikalisch polymerisiert werden, und gegebenenfalls die Monomere vor der Polymerisation oder das Polymer nach der Polymerisation mit Ammoniak oder organischen Aminen oder einer Li⁺-, Na⁺-, K⁺-, Ca⁺⁺-, Mg⁺⁺-, Zn⁺⁺- oder Al⁺⁺⁺-enthaltenden Base, vorzugsweise mit den entsprechenden Hydroxiden oder Carbonaten und besonders bevorzugt mit Hydroxiden, neutralisiert werden.

Radikalische Polymerisationen sind dem Fachmann allgemein bekannt und in Standardwerken der Literatur, z. B. in "Makromolekulare Chemie: Eine Einführung" von Bernd Tieke, Wiley-VCH, 2. vollständig überarbeitete und erweiterte Auflage (9. September 2005) ISBN-10: 3527313796 ausführlich beschrieben.

Die erfindungsgemäßen Polymere zeichnen sich durch eine gute Hautmilde und ein angenehmes Hautgefühl aus. Die erfindungsgemäßen Polymere sind darüber hinaus säurestabil. Da die erfindungsgemäßen Polymere auch bei sauren pH-Werten verdicken, kann man verdickte kosmetische Produkte vorteilhafterweise auch mit organischen Säuren, wie Benzoesäure, Sorbinsäure, Paramethoxybenzoesäure konservieren, da auch bei den notwendigen niedrigen pH-Werten genügend Verdickerleistung zur Verfügung steht. Mit ihnen können klare Lösungen erhalten werden.

Die erfindungsgemäßen Polymere eignen sich zudem in vorteilhafter Weise zur Herstellung von kosmetischen, dermatologischen oder pharmazeutischen Zusammensetzungen.

Weiterer Gegenstand der vorliegenden Erfindung ist daher die Verwendung eines oder mehrerer erfindungsgemäßer Polymere zur Herstellung von kosmetischen, dermatologischen oder pharmazeutischen Zusammensetzungen sowie kosmetische, dermatologische oder pharmazeutische Zusammensetzungen enthaltend ein oder mehrere erfindungsgemäße Polymere.

Die erfindungsgemäßen Zusammensetzungen enthalten, bezogen auf die fertigen Zusammensetzungen, vorzugsweise 0,01 bis 10,0 Gew.-%, besonders bevorzugt 0,1 bis 5,0 Gew.-% und insbesondere bevorzugt 0,5 bis 2,0 Gew.-% an den erfindungsgemäßen Polymeren.

In einer bevorzugten Ausführungsform der Erfindung haben die erfindungsgemäßen tensidhaltigen Zusammensetzungen Viskositäten vorzugsweise im Bereich von 100 bis 50.000 mPa · s, besonders bevorzugt im Bereich von 100 bis 10.000 mPa · s, insbesondere bevorzugt im Bereich von 500 bis 5.000 mPa · s und außerordentlich bevorzugt im Bereich von 500 bis 4.000 mPa · s (25 °C, Brookfield RVT, T-C Spindel bei 5 rpm).

In einer weiteren bevorzugten Ausführungsform der Erfindung haben die erfindungsgemäßen Zusammensetzungen, wie Cremes, Lotions, wässrige und wässrige alkoholische Formulierungen Viskositäten vorzugsweise im Bereich von 100 bis 50.000 mPa · s, besonders bevorzugt im Bereich von 500 bis 30.000 mPa · s und insbesondere bevorzugt im Bereich von 1000 bis 20.000 mPa · s (25 °C, Brookfield RVT, T-C Spindel bei 5 rpm).

In einer weiteren bevorzugten Ausführungsform der Erfindung liegen die erfindungsgemäßen Zusammensetzungen in Form von Fluids, Gelen, Ölen, Schäumen, Sprays, Lotions oder Cremes vor.

Die erfindungsgemäßen Zusammensetzungen liegen vorzugsweise auf wässriger oder wässrig-alkoholischer Basis oder als Öl-in-Wasser Emulsionen vor.

In einer besonders bevorzugten Ausführungsform der Erfindung liegen die erfindungsgemäßen Zusammensetzungen auf wässriger oder wässrig-alkoholischer Basis oder als Öl-in-Wasser Emulsionen vor, insbesondere bevorzugt als wässrige tensidhaltige, wässrig-alkoholische tensidhaltige Zusammensetzungen, und enthalten vorzugsweise, bezogen auf das Gesamtgewicht der Zusammensetzungen,
a) bis zu 98,0 Gew.-%, vorzugsweise 60,0 bis 98,0 Gew.-%, besonders bevorzugt 70,0 bis 94,4 Gew.-%, insbesondere bevorzugt 75,0 bis 94,5 Gew.-% einer Wasserphase oder wässrigen-alkoholischen Phase,
b) bis zu 50,0 Gew.-%, vorzugsweise 1,0 bis 30,0 Gew.-%, besonders bevorzugt 5,0 bis 20,0 Gew.-%, insbesondere bevorzugt 5,0 bis 15,0 Gew.-% eines oder mehrerer Tenside,
c) bis zu 10,0 Gew.%, vorzugsweise 0,01 bis 10,0 Gew.-%, besonders bevorzugt 0,1 bis 5,0 Gew.%, insbesondere bevorzugt 0,5 bis 2,0 Gew.-% an einem oder mehreren der erfindungsgemäßen Polymere und
d) bis zu 50,0 Gew.-%, vorzugsweise 0,5 bis 38,99 Gew.-%, besonders bevorzugt 0,5 bis 6,0 Gew.-%, insbesondere bevorzugt 1,0 bis 5,0 Gew.-% eines oder mehrerer weiterer Zusatzstoffe, wobei der eine oder die mehreren Zusatzstoffe insbesondere ein oder mehrerer Öle bedeuten, wenn die erfindungsgemäße Zusammensetzung als ÖI-in-Wasser Emulsionen vorliegen.

Für die erfindungsgemäßen Zusammensetzungen auf wässrig-alkoholischer oder auch alkoholischer Basis kommen alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol oder Glycerin sowie Alkylenglykole, insbesondere Propylen-, Butylen- oder Hexylenglykol, und Mischungen aus den genannten Alkoholen. Weitere bevorzugte Alkohole sind Polyethylenglykole mit einer relativen Molekülmasse unter 2.000. Insbesondere ist ein Einsatz von Polyethylenglykol mit einer relativen Molekülmasse zwischen 200 und 600 und von Polyethylenglykol mit einer relativen Molekülmasse zwischen 400 und 600 bevorzugt.

Die erfindungsgemäßen Zusammensetzungen können ein oder mehrere Öle enthalten.

Die Öle können vorteilhafterweise ausgewählt werden aus den Gruppen der Triglyceride, natürlichen und synthetischen Fettkörper, vorzugsweise Ester von Fettsäuren mit Alkoholen niedriger C-Zahl, z. B. mit Methanol, Isopropanol, Propylenglykol oder Glycerin, oder Ester von Fettalkoholen mit Alkansäuren niedriger C-Zahl oder mit Fettsäuren oder aus der Gruppe der Alkylbenzoate, sowie natürlichen oder synthetischen Kohlenwasserstoffölen.

In Betracht kommen Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol^{®} 318 sowie das Handelsprodukt Velsan^{®} CCT (Capryl-Caprinsäure-triglycerid, Clariant). Auch gehärtete Triglyceride sind erfindungsgemäß bevorzugt. Auch Öle tierischen Ursprungs, beispielsweise Rindertalg, Perhydrosqualen, Lanolin können eingesetzt werden.

Eine weitere Klasse von bevorzugten Ölkörpern sind die Benzoesäureester von linearen oder verzweigten C₈₋₂₂-Alkanolen, z. B. die Handelsprodukte Finsolv^{®} SB (Isostearylbenzoat), Finsolv^{®} TN (C₁₂-C₁₅-Alkylbenzoat) und Finsolv^{®}EB (Ethylhexylbenzoat).

Eine weitere Klasse von bevorzugten Ölkörpern sind die Dialkylether mit insgesamt 12 bis 36 Kohlenstoffatomen, insbesondere mit 12 bis 24 Kohlenstoffatomen, wie z. B. Di-n-octylether (Cetiol^{®} OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether, Di-3-ethyldecylether, tert.-Butyl-n-octylether, iso-Pentyl-n-octylether und 2-Methyl-pentyl-n-octylether sowie Di-tert.-butylether und Di-iso-pentylether.

Ebenso in Betracht kommen verzweigte gesättigte oder ungesättigte Fettalkohole mit 6 - 30 Kohlenstoffatomen, z. B. Isostearylalkohol, sowie Guerbetalkohole.

Eine weitere Klasse von bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z. B. unter dem Handelsnamen Cosmacol^{®} der EniChem, Augusta Industriale.

Eine weitere Klasse von bevorzugten Ölkörpern sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylglycoldicaprylat sowie Di-isotridecylacelaat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol^{®} CC).

Eine weitere Klasse von bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von bevorzugten Ölkörpern sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl^{®}-Serie, Squalan, Squalen, und alicyclische Kohlenwasserstoffe, z. B. das Handelsprodukt 1,3-Di-(2-ethyl-hexyl)-cyclohexan (Cetiol^{®}S), Ozokerit, und Ceresin.

Ebenso in Betracht kommen Silikonöle bzw. -wachse, vorzugsweise Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare^{®} Silicone 41 M65, SilCare^{®} Silicone 41 M70, SilCare^{®} Silicone 41 M80 (Clariant) erhältlichen Dimethicone, Stearyldimethylpolysiloxan, C₂₀-C₂₄-Alkyl-dimethylpolysiloxan, C₂₄-C₂₈-Alkyl-dimethylpolysiloxan, aber auch die unter SilCare^{®} Silicone 41 M40, SilCare^{®} Silicone 41 M50 (Clariant) erhältlichen Methicone, weiterhin Trimethylsiloxysilicate [(CH₂)₃SiO)_{½}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Coming Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Die erfindungsgemäßen Zusammensetzungen enthalten vorzugsweise ein oder mehrere Tenside.

Als Tenside werden im Rahmen der vorliegenden Erfindung Substanzen bezeichnet, die die Oberflächenspannung einer Flüssigkeit oder die Grenzflächenspannung zwischen zwei Phasen herabsetzen und die Bildung von Dispersionen oder Emulsionen ermöglichen oder unterstützen. Dies bedeutet insbesondere, dass der Begriff "Tenside" im Rahmen der vorliegenden Erfindung auch Substanzen umfasst, die üblicherweise als Emulgatoren bezeichnet werden.

Die Tenside können vorteilhafterweise ausgewählt werden aus den Gruppen der nichtionischen Tenside, anionischen Tenside, kationischen Tenside, amphotheren Tenside und Betaintenside.

Die Menge der in den erfindungsgemäßen Zusammensetzungen (z. B. im Falle von Rinse-Off-Produkten) enthaltenen Tenside beträgt, bezogen auf die fertigen erfindungsgemäßen Zusammensetzungen, vorzugsweise von 0,01 bis 50,0 Gew.-%, besonders bevorzugt von 1,0 bis 30,0 Gew.%, insbesondere bevorzugt von 5,0 bis 20,0 Gew.-% und außerordentlich bevorzugt von 5,0 bis 15,0 Gew.%.

Als anionische Tenside bevorzugt sind (C₁₀-C₂₂)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholphosphate, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate und Acylglycinate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Die Menge der anionischen Tenside in den erfindungsgemäßen Zusammensetzungen beträgt bevorzugt von 0,1 bis 30,0 Gew.-%, besonders bevorzugt von 0,2 bis 20,0 Gew.-% und insbesondere bevorzugt von 0,5 bis 15,0 Gew.-%, bezogen auf die fertigen Zusammensetzungen.

Bevorzugte kationische Tenside sind quartäre Ammonium-Salze, wie Di-(C₈-C₂₂)-Alkyl-dimethylammoniumchlorid oder -bromid, vorzugsweise Di-(C₈-C₂₂)-Alkyl-dimethylammoniumchlorid oder -bromid; (C₈-C₂₂)-Alkyl-dimethyl-ethylammoniumchlorid oder -bromid; (C₈-C₂₂)-Alkyl-trimethylammoniumchlorid oder -bromid, vorzugsweise Cetyltrimethylammoniumchlorid oder -bromid und (C₈-C₂₂)Alkyl-trimethylammoniumchlorid oder -bromid; (C₁₀-C₂₄)-Alkyl-dimethylbenzyl-ammoniumchlorid oder -bromid, vorzugsweise (C₁₂-C₁₈)-Alkyl-dimethylbenzyl-ammoniumchlorid, (C₈-C₂₂)-Alkyl-dimethyl-hydroxyethylammoniumchlorid, -phosphat, -sulfat, -lactat, (C₈-C₂₂)-Alkylamidopropyltrimethylammoniumchlorid, -methosulfat, N,N-bis(2-C₈-C₂₂-Alkanoyl-oxyethyl)-dimethylammoniumchlorid, -methosulfat, N,N-bis(2-C₈-C₂₂-Alkanoyl-oxyethyl)hydroxyethyl-methyl-ammoniumchlorid, -methosulfat.

Die Menge der kationischen Tenside in den erfindungsgemäßen Zusammensetzungen beträgt bevorzugt von 0,1 bis 10,0 Gew.-%, besonders bevorzugt von 0,5 bis 7,0 Gew.-% und insbesondere bevorzugt von 1,0 bis 5,0 Gew.-%, bezogen auf die fertigen Formulierungen.

Als nichtionische Tenside bevorzugt sind Fettalkoholethoxylate (Alkylpolyethylenglykole); Alkylphenolpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäurealkanolamide, (Fettsäureamidpolyethylenglykole); Saccharoseester; Sorbitester und Sorbitanester und deren Polyglykolether, sowie C₈-C₂₂-Alkylpolyglucoside.

Die Menge der nichtionischen Tenside in den erfindungsgemäßen Zusammensetzungen (z. B. im Falle von Rinse-off-Produkten) liegt bevorzugt im Bereich von 1,0 bis 20,0 Gew.-%, besonders bevorzugt von 2,0 bis 10,0 Gew.-% und insbesondere bevorzugt von 3,0 bis 7,0 Gew.-%, bezogen auf die fertigen Zusammensetzungen.

Weiterhin können die erfindungsgemäßen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-, Sulfat - oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide, Fettsäureamidoalkyl-dimethylaminoxid.

Eine weitere bevorzugte Gruppe von Tensiden sind Betaintenside, auch zwitterionische Tenside genannt. Diese enthalten im selben Molekül eine kationische Gruppe, insbesondere eine Ammonium-Gruppe und eine anionische Gruppe, die eine Carboxylat-Gruppe, Sulfat-Gruppe oder Sulfonat-Gruppe sein kann. Geeignete Betaine sind vorzugsweise Alkylbetaine wie Coco-Betain oder Fettsäurealkylamidopropylbetaine, beispielsweise Kokosacylamidopropyldimethylbetain oder die C₁₂- bis C₁₈-Dimethylaminohexanoate bzw. die C₁₀- bis C₁₈-Acylamidopropandimetylbetaine.

Die Menge der amphoteren Tenside und/oder Betaintenside in den erfindungsgemäßen Zusammensetzungen beträgt bevorzugt von 0,5 bis 20,0 Gew.-% und besonders bevorzugt von 1,0 bis 15,0 Gew.-%, bezogen auf die fertigen Zusammensetzungen.

Bevorzugte Tenside sind Laurylsulfat, Laurethsulfat, Cocoamidopropylbetain, Alkylbetaine wie Coco-Betain, Natriumcocoylglutamat und Lauroamphoacetat.

Besonders bevorzugte Tenside sind alkylierte Ethersulfate mit einer linearen oder verzweigten Alkylgruppe mit 1 bis 30 Kohlenstoffatomen, einer linearen oder verzweigten einfach oder mehrfach ungesättigten Alkenylgruppe mit 2 bis 30 Kohlenstoffatomen, Betaine und deren Derivate und insbesondere bevorzugt sind alkylierte Ethersulfate mit einer linearen oder verzweigten Alkylgruppe mit 12 bis 22 Kohlenstoffatomen, Betaine und deren Derivate und deren Mischungen.

In einer weiteren bevorzugten Ausführungsform der Erfindung enthalten die erfindungsgemäßen Zusammensetzungen zusätzlich noch als schaumverstärkende Mittel Co-Tenside aus der Gruppe der Alkylbetaine, Alkylamidobetaine, Aminopropionate, Aminoglycinate, Imidazoliniumbetaine und Sulfobetaine, Aminoxide, Fettsäurealkanolamide und Polyhydroxyamide.

Die erfindungsgemäßen Zusammensetzungen können als weitere Hilfs- und Zusatzstoffe beispielsweise Wachse, Emulgatoren, Co-Emulgatoren, Solubilisatoren, Elektrolyte, Hydroxysäuren, Stabilisatoren, kationische Polymere, Filmbildner, weitere Verdicker, Gelierungsmittel, Überfettungsmittel, Rückfetter, antimikrobielle Wirkstoffe, biogene Wirkstoffe, Adstringentien, deodorierende Stoffe, Sonnenschutzfilter, Antioxidantien, Feuchthaltemittel, Lösungsmittel, Farbmittel, Perlglanzmittel, Duftstoffe, Trübungsmittel und/oder Silikone enthalten.

Die erfindungsgemäßen Zusammensetzungen können Wachse, beispielsweise Paraffinwachse, Mikrowachse und Ozokerite, Bienenwachs und ihre Teilfraktionen sowie der Bienenwachsderivate, Wachse aus der Gruppe der homopolymeren Polyethylene oder Coplymere der α-Olefine, sowie natürliche Wachse wie Reiswachs, Candellilawachs, Carnaubawachs, Japanwachs oder Schellackwachs enthalten.

Als Emulgatoren, Co-Emulgatoren und Solubilisatoren können nichtionische, anionische, kationische oder amphotere oberflächenaktive Verbindungen eingesetzt werden.

Als nichtionogene oberflächenaktive Verbindungen kommen vorzugsweise in Betracht:
Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono - und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z. B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Besonders bevorzugt zum Einsatz kommen Fettalkoholethoxylate, gewählt aus der Gruppe der ethoxylierten Stearylalkohole, Isostearylalkohole, Cetylalkohole, Isocetylalkohole, Oleylalkohole, Laurylalkohole, Isolaurylalkohole und Cetylstearylalkohole, insbesondere Polyethylenglycol(13)stearylether, Polyethylenglycol(14)stearylether, Polyethylenglycol(15)stearylether, Polyethylenglycol(16)stearylether, Polyethylenglycol(17)stearylether, Polyethylenglycol(18)stearylether, Polyethy(englycol(19)stearylether, Polyethylenglycol(20)stearylether, Polyethylenglycol(12)isostearylether, Polyethylenglycol(13)isostearylether, Polyethylenglycol(14)isostearylether, Polyethylenglycol(15)isostearylether, Polyethylenglycol(16)isostearylether, Polyethylenglycol(17)isostearylether, Polyethylenglycol(18)isostearylether, Polyethylenglycol(19)isostearylether, Polyethylenglycol(20)isostearylether, Polyethylenglycol(13)cetylether, Polyethylenglycol(14)cetylether, Polyethylenglycol(15)cetylether, Polyethylenglycol(16)cetylether, Polyethylenglycol(17)cetylether, Polyethylenglycol(18)cetylether, Polyethylenglycol(19)cetylether, Polyethylenglycol(20)cetylether, Polyethylenglycol(13)isocetylether, Polyethylenglycol(14)isocetylether, Polyethylenglycol(15)isocetylether, Polyethylenglycol(16)isocetylether, Polyethylenglycol(17)isocetylether, Polyethylenglycol(18)isocetylether, Polyethylenglycol(19)isocetylether, Polyethylenglycol(20)isocetylether, Polyethylenglycol(12)oleylether, Polyethylenglycol(13)oleylether, Polyethylenglycol(14)oleylether, Polyethylenglycol(15)oleylether, Polyethylenglycol(12)taurylether, Polyethylenglycol(12)isolaurylether, Polyethylenglycol(13)cetylstearylether, Polyethylenglycol(14)cetylsteärylether, Polyethylenglycol(15)cetylstearylether, Polyethylenglycol(16)cetylstearylether, Polyethylenglycol(17)cetylstearylether, Polyethylenglycol(18)cetylstearylether, Polyethylenglycol(19)cetylstearylether.

Ebenso bevorzugt sind Fettsäureethoxylate, gewählt aus der Gruppe der ethoxylierten Stearate, Isostearate und Oleate, insbesondere Polyethylenglycol(20)stearat, Polyethylenglykol(21)stearat, Polyethylenglykol(22)stearat, Polyethylenglykol(23)stearat, Polyethylenglykol(24)stearat, Polyethylenglykol(25)stearat, Polyethylenglykol(12)isostearat, Polyethylenglykol(13)isostearat, Polyethylenglykol(14)isostearat, Polyethylenglykol(15)isostearat, Polyethylenglykol(16)isostearat, Polyethylenglykol(17)isostearat, Polyethylenglykol(18)isostearat, Polyethylenglykol(19)isostearat, Polyethylenglykol(20)isostearat, Polyethylenglykol(21)isostearat, Polyethylenglykol(22)isostearat, Polyethylenglykol(23)isostearat, Polyethylenglykol(24)isostearat, Polyethylenglykol(25)isostearat, Polyethylenglykol(12)oleat, Polyethylenglykol(13)oleat, Polyethylenglykol(14)oleat, Polyethylenglykol(15)oleat, Polyethylenglykol(16)oleat, Polyethylenglykol(17)oleat, Polyethylenglykol(18)oleat, Polyethylenglykol(19)oleat, Polyethylenglykol(20)oleat.

Als ethoxylierte Alkylethercarbonsäure oder deren Salze kann vorteilhafterweise das Natrium Laureth-11-carboxylat verwendet werden.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat,
Polyethylenglykol(6)glycerylcaprat/caprinat, Polyethylenglykol(20)glyceryloleat, Polyethylenglykol(20)glycerylisostearat und
Polyethylenglykol(18)glyceryloleat/cocoat zu wählen.

Unter den Sorbitanestern eignen sich besonders
Polyethylenglykol(20)sorbitanmonolaurat,
Polyethylenglycol(20)sorbitanmonostearat,
Polyethylenglycol(20)sorbitanmonoisostearat,
Polyethylenglycol(20)sorbitanmonopalmitat,
Polyethylenglycol(20)sorbitanmonooleat.

Besonders vorteilhafte Coemulgatoren sind Glycerylmonostearat, Glycerylmonooleat, Diglycerylmonostearat, Glycerylisostearat, Polyglyceryl-3-oleat, Polyglyceryl-3-diisostearat, Polyglyceryl-4-isostearat, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat, Sorbitanmonoisostearat, Sorbitanstearat, Sorbitanoleat, Saccharosedistearat, Lecithin, PEG-7-hydriertes Ricinusöl, Cetylalkohol, Stearylalkohol, Behenylalkohol, Isobehenylalkohol und Polyethylenglycol(2)stearylether (Steareth-2), Alkylmethiconcopolyole und Alkyl-Dimethiconcopolyole, insbesondere Cetyldimethiconcopolyol, Laurylmethiconcopolyol.

Die erfindungsgemäßen Zusammensetzungen können einen oder mehrere der Emulgatoren, Co-Emulgatoren oder Solubilisatoren in Mengen von 0,1 bis 20,0 Gew.-%, vorzugsweise 1,0 bis 15,0 Gew.-% und besonders bevorzugt 3,0 bis 10,0 Gew.-%, bezogen auf die fertigen Zusammensetzungen, enthalten.

Als Elektrolyt zum Einsatz können kommen anorganische Salze, bevorzugt Ammonium- oder Metallsalze, besonders bevorzugt von Halogeniden, beispielsweise CaCl₂, MgCl₂, LiCl, KCl und NaCl, Carbonaten, Hydrogencarbonaten, Phosphaten, Sulfaten, Nitraten, insbesondere bevorzugt Natriumchlorid, und/oder organische Salze, bevorzugt Ammonium- oder Metallsalze, besonders bevorzugt der Glykolsäure, Milchsäure, Zitronensäure, Weinsäure, Mandelsäure, Salicylsäure, Ascorbinsäure, Brenztraubensäure, Fumarsäure, Retinoesäure, Sulfonsäuren, Benzoesäure, Kojisäure, Fruchtsäure, Äpfelsäure, Gluconsäure oder Galacturonsäure.
Hierzu zählen auch Aluminiumsalze, bevorzugt Aluminiumchlorohydrat oder Aluminium-Zirkonium-Komplexsalze.

In einer bevorzugten Ausführungsform der Erfindung enthalten die erfindungsgemäßen Zusammensetzungen daher ein oder mehrere Substanzen ausgewählt aus anorganischen und organischen Salzen.

Als Elektrolyt können die erfindungsgemäßen Zusammensetzungen auch Mischungen verschiedener Salze enthalten. Der Gehalt an dem einen oder den mehreren Elektrolyten, bezogen auf die gesamte erfindungsgemäße Zusammensetzung ist vorzugsweise von 0,01 bis 20,0 Gew.-%, besonders bevorzugt von 0,1 bis 10,0 Gew.-% und insbesondere bevorzugt von 0,5 bis 5,0 Gew.-%.

Die erfindungsgemäßen Polymere sind säurestabil und eignen sich vorzugsweise zur Verwendung in kosmetischen, pharmazeutischen und/oder dermatologischen Zusammensetzungen mit niedrigem pH-Wert von 2 bis 6, insbesondere für Produkte zur Haar- und Hautreinigung und zur Körperpflege.

Die Verwendung von sauren Additiven und deren Salzen macht es teilweise notwendig, den pH-Wert der kosmetischen oder dermatologischen Zusammensetzungen in einen deutlich sauren Bereich einzustellen.

In einer weiteren bevorzugten Ausführungsform der Erfindung enthalten die erfindungsgemäßen Zusammensetzungen ein oder mehrere Hydroxysäuren, besonders bevorzugt ein oder mehrere Substanzen ausgewählt aus alpha- und beta-Hydroxysäuren.

An Hydroxysäuren können die erfindungsgemäßen Zusammensetzungen vorzugsweise Milchsäure, Glykolsäure, Salicylsäure und alkylierte Salicylsäuren oder Zitronensäure enthalten. Weiterhin können erfindungsgemäße Formulierungen weitere saure Komponenten enthalten. Als Wirkstoff in Betracht kommen Weinsäure, Mandelsäure, Caffeic acid, Brenztraubensäure, Oligooxa Mono- und Dicarbonsäuren, Fumarsäure, Retinoesäure, Sulfonsäuren, Benzoesäure, Kojisäure, Fruchtsäure, Äpfelsäure, Gluconsäure, Pyruvic Acid, Galacturonic Acid, Ribonic Acid, und all deren Derivate, Polyglykoldisäuren in freier oder teilweiser neutralisierter Form, Vitamin C (Ascorbinsäure), Vitamin C-Derivate, Dihydroxyaceton oder Skin-whitening Actives wie Arbutin oder Glycyrrhetinsäure und deren Salze. Der Gehalt an einer oder mehreren dieser soeben genannten Substanzen, bezogen auf die gesamte erfindungsgemäße Zusammensetzung, ist vorzugsweise von 0,1 bis 20,0 Gew.-%, besonders bevorzugt von 0,2 bis 10,0 Gew.-% und insbesondere bevorzugt von 0,5 bis 5,0 Gew.%.

In einer weiteren bevorzugten Ausführungsform der Erfindung enthalten die erfindungsgemäßen Zusammensetzungen daher ein oder mehrere Substanzen ausgewählt aus Vitamin C und Vitamin C-Derivaten, wobei die Vitamin C-Derivate vorzugsweise ausgewählt sind aus Natriumascorbylphosphat, Magnesiumascorbylphoshat und Magnesiumascorbylglucosid.

In einer weiteren bevorzugten Ausführungsform der Erfindung enthalten die erfindungsgemäßen Zusammensetzungen eine oder mehrere Substanzen ausgewählt aus Benzoesäure, Sorbinsäure, Salicylsäure, Milchsäure und Paramethoxybenzoesäure. Dadurch, dass die erfindungsgemäßen Polymere auch im sauren pH-Bereich verdicken und eine Fließgrenze aufbauen, kann man mit den oben genannten organischen Säuren als Konservierer arbeiten.

Zu den erfindungsgemäßen Copolymeren können als zusätzliche Stabilisatoren Metallsalze von Fettsäuren, wie z. B. Magnesium-, Aluminium- und/oder Zinkstearat eingesetzt werden, bevorzugt in Mengen von 0,1 bis 10,0 Gew.-%, vorzugsweise von 0,5 bis 8,0 Gew.-% und besonders bevorzugt von 1,0 bis 5,0 Gew.-%, bezogen auf die fertigen Zusammensetzungen.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guarhydroxypropyltriammonium-chloride, sowie Calciumalginat und Ammoniumalginat. Des Weiteren können eingesetzt werden kationische Cellulosederivate; kationische Stärke; Copolymere von Diallylammoniumsalzen und Acrylamiden; quatemierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxypropyldiethylentriamin; Polyaminopolyamid und kationische Chitinderivate, wie beispielsweise Chitosan.

Die erfindungsgemäßen Zusammensetzungen können einen oder mehrere der oben genannten kationischen Polymere in Mengen von 0,1 bis 5,0 Gew.-%, vorzugsweise von 0,2 bis 3,0 Gew.-% und besonders bevorzugt von 0,5 bis 2,0 Gew.-%, bezogen auf die fertigen Zusammensetzungen, enthalten.

Des Weiteren können die erfindungsgemäßen Zusammensetzungen Filmbildner enthalten, die je nach Anwendungszweck ausgewählt sind aus Salzen der Phenylbenzimidazolsulfonsäure, wasserlöslichen Polyurethanen, beispielsweise C₁₀-Polycarbamylpolyglycerylester, Polyvinylalkohol, Polyvinylpyrrolidoncopolymeren, beispielsweise Vinylpyrrolidon/Vinylacetatcopolymer, wasserlöslichen Acrylsäurepolymeren/Copolymeren bzw. deren Estern oder Salzen, beispielsweise Partialestercopolymere der Acryl/Methacrylsäure, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex^{®} A 60 (Clariant) erhältlich.

Die erfindungsgemäßen Zusammensetzungen können einen oder mehrere Filmbildner in Mengen von 0,1 bis 10,0 Gew.-%, vorzugsweise von 0,2 bis 5,0 Gew.% und besonders bevorzugt von 0,5 bis 3,0 Gew.-%, bezogen auf die fertigen Zusammensetzungen, enthalten.

Die gewünschte Viskosität der Zusammensetzungen kann durch Zugabe von weiteren Verdickern und Gelierungsmittel eingestellt werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammoniumacryloyldimethyltaurate/VP-Copolymer Verwendung finden.

In einer weiteren bevorzugten Ausführungsform der Erfindung enthalten die erfindungsgemäßen Zusammensetzungen 0,01 bis 20,0 Gew.-%, besonders bevorzugt 0,1 bis 10,0 Gew.-%, insbesondere bevorzugt 0,2 bis 3,0 Gew.-% und ganz besonders bevorzugt 0,4 bis 2,0 Gew.-% an Verdickern bzw. Geliermitteln, bezogen auf die fertigen erfindungsgemäßen Zusammemnsetzungen.

Als Überfettungsmittel oder Rückfetter können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt werden, bezogen auf die fertigen erfindungsgemäßen Formulierungen.

An antimikrobiellen Wirkstoffen können Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox^{®}, lodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl - und Propylparaben, sowie deren Na-Salze, Pentandiol 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz kommen.

Die erfindungsgemäßen Zusammensetzungen enthalten die antimikrobiellen Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen erfindungsgemäßen Zusammensetzungen.

Die erfindungsgemäßen Zusammensetzungen können des Weiteren biogene Wirkstoffe, ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol^{®}, Allantoin, Phytantriol^{®}, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Die erfindungsgemäßen Zusammensetzungen können biogene Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen Zusammensetzungen, enthalten.

Die erfindungsgemäßen Zusammensetzungen können Adstringentien, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink, sowie Aluminiumchlorohydrate bevorzugt in Mengen von 0 bis 50,0 Gew.-%, besonders bevorzugt in Mengen von 0,01 bis 10,0 Gew.-% und insbesondere bevorzugt in Mengen von 0,1 bis 10,0 Gew.-% enthalten.

Als deodorierende Stoffe bevorzugt sind Allantoin und Bisabolol. Diese werden vorzugsweise in Mengen von 0,0001 bis 10,0 Gew.-% eingesetzt.

In einer weiteren bevorzugten Ausführungsform der Erfindung enthalten die erfindungsgemäßen Zusammensetzungen ein oder mehrere Substanzen ausgewählt aus anorganischen und organischen UV-Filtern und liegen besonders bevorzugt in der Form einer Sonnenschutzzusammensetzung vor.

Die erfindungsgemäßen Zusammensetzungen können als Pigmente/Mikropigmente sowie als anorganische Sonnenschutzfilter bzw. UV-Filter mikrofeines Titandioxid, Glimmer-Titanoxid, Eisenoxide, Glimmer-Eisenoxid, Zinkoxid, Siliciumoxide, Ultramarinblau oder Chromoxide enthalten.

Die organischen Sonnenschutzfilter bzw. UV-Filter sind vorzugsweise ausgewählt aus 4-Aminobenzoesäure, 3-(4'-Trimethylammonium)-benzyliden-boran-2-on-methylsulfat, Camphor Benzalkonium Methosulfat, 3,3,5-Trimethylcyclohexylsalicylat, 2-Hydroxy-4-methoxybenzophenon, 2-Phenylbenzimidazol-5-sulfonsäure und ihre Kalium-, Natrium- und Triethanolaminsalze, 3,3'-(1,4-Phenylendimethin)-bis-(7,7-dimethyl-2-oxobicyclo[2.2.1]-heptan-1-methansulfonsäure) und ihre Salze, 1-(4-tert.-Butylphenyl)-3-(4-methoxyphenyl)propan-1,3-dion, 3-(4'-Sulfo)-benzyliden-bornan-2-on und seine Salze, 2-Cyan-3,3-diphenyl-acrylsäure-(2-ethylhexylester), Polymere von N-[2(und 4)-(2-oxoborn-3-ylidenmethyl)benzyl]-acrylamid, 4-Methoxy-zimtsäure-2-ethylhexylester, ethoxyliertes Ethyl-4-amino-benzoat, 4-Methoxy-zimtsäureisoamylester, 2,4,6-Tris-[p-(2-ethylhexyloxycarbonyl)anilino]-1,3,5-triazin, 2-(2H-benzotriazol-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-aminocarbonyl)phenylamino]-1,3,5-triazin-2,4-yl)diimino]bis-(benzoesäure-2-ethylhexylester), Benzophenon-3, Benzophenon-4 (Säure), 3-(4'-Methylbenzyliden)-D,L-Campher, 3-Benzyliden-Campher, Salicylsäure-2-ethylhexylester, 4-Dimethylaminobenzoesäure-2-ethylhexylester, Hydroxy-4-methoxy-benzophenon-5-sulfonsäure (Sulfisobenzonum) und das Natriumsalz, 4-Isopropylbenzylsalicylat, N,N,N-Trimethyl-4-(2-oxoborn-3-ylidenemethyl) anilium methyl sulphate, Homosalate (INN), Oxybenzone (INN), 2-Phenylbenzimidazole-5-sulfonsäure und ihre Natrium-, Kalium-, und Triethanolaminsalze, Octylmethoxyzimtsäure, Isopentyl-4-methoxyzimtsäure, Isoamyl-p-methoxyzimtsäure, 2,4,6-Trianilino-(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometrizole Trisiloxane) benzoesäure, 4,4-((6-(((1,1-dimethylethyl)amino)carbonyl)phenyl)amino)-1,3,5-triazine-2,4-diyl)diimino)bis,bis(2-ethylhexyl)ester) benzoesäure, 4,4-((6-(((1,1-dimethylethyl)amino)-carbonyl)phenyl)amino)-1,3,5-triazine-2,4-diyl)diimino)bis,bis(2-ethylhexyl)ester), 3-(4'-Methylbenzyliden)-D,L-campher (4-Methylbenzyliden Camphor), Benzyliden-camphor-sulfonsäure, Octocrylen, Polyacrylamidomethyl-Benzyliden-Camphor, 2-Ethylhexyl salicylat (Octyl Salicylat), 4-Dimethyl-aminobenzoesäureethyl-2-hexylester (octyl dimethyl PABA), PEG-25 PABA, 2-Hydroxy-4-methoxybenzo-phenone-5-sulfonsäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat, Di-p-methoxyzimtsäure, p-Amino-benzoesäure und deren Ester, 4-tert-Butyl-4'-methoxydibenzoylmethan, 4-(2-β-Glucopyranoxy)propoxy-2-hydroxybenzophenon, Octyl Salicylat, Methyl-2,5-diisopropylzimtsäure, Cinoxat, Dihydroxy-dimethoxybenzophenon, Dinatriumsalz von 2,2'-Dihydroxy-4,4'-dimethoxy-5,5'-disulfobenzophenon, Dihydroxybenzophenon, 1,3,4-Dimethoxyphenyl-4,4-dimethyl-1,3-pentanedion, 2-Ethylhexyl-dimethoxybenzyliden-dioxoimidazolidinpropionat, Methylen-Bis-Benztriazolyl Tetramethylbutylphenol, Phenyldibenzimidazoltetrasulfonat, Bis-Ethylhexyloxyphenol-Methoxyphenol-Triazin, Tetrahydroxybenzophenone, Terephthalylidendicampher-sulfonsäure, 2,4,6-tris[4,2-Ethylhexyloxycarbonyl)anilino]-1,3,5-triazin, Methyl-bis(trimethylsiloxy)silyl-isopentyl trimethoxy-zimtsäure, Amyl-p-dimethylaminobenzoat, Amyl-p-dimethylamino benzoat, 2-Ethylhexyl-p-dimethylaminobenzoat, Isopropyl-p-methoxyzimtsäure/ Diisopropylzimtsäureester, 2-Ethylhexyl-p-methoxyzimtsäure, 2-Hydroxy-4-methoxy benzophenon, 2-Hydroxy-4-methoxybenzophenon-5-sulfsäure und das Trihydrat, sowie 2-Hydroxy-4-methoxybenzophenon-5-sulfonat Natriumsalz und Phenyl-benzimidazol-sulfonsäure.

Die Menge der vorgenannten Sonnenschutzfilter (eine oder mehrere Verbindungen) in den erfindungsgemäßen Zusammensetzungen beträgt vorzugsweise von 0,001 bis 30,0 Gew.-%, besonders bevorzugt von 0,05 bis 20,0 Gew.-% und insbesondere bevorzugt von 1,0 bis 10,0 Gew.-%, bezogen auf das Gesamtgewicht der fertigen Zusammensetzung.

Die erfindungsgemäßen Zusammensetzungen können einen oder mehrere Antioxidantien enthalten, vorzugsweise ausgewählt aus Aminosäuren (z. B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Camosin und deren Derivaten (z. B. Anserin), Carotinoiden, Carotinen (z. B. α-Carotin, β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (z. B. Ester, Ether, Peptide, Lipide, Nukleotide, Nukleoside und Salze), sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Gallensäure, Gallenextrakten, Bilirubin, Biliverdin, EDTA, EGTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin E-acetat), Vitamin A und Derivaten (Vitamin A-palmitat) sowie Koniferylbenzoat des Benzoeharzes, Rutinsäure und deren Derivaten, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, ZnSO₄), Selen und dessen Derivaten (z. B. Selenmethionin), Stilbenen und deren Derivaten (z. B. Stilbenoxid, trans-Stilbenoxid), Superoxid-Dismutase und den erfindungsgemäß geeigneten Derivaten (Salzen, Estern, Ethern, Zuckern, Nukleotiden, Nukleosiden, Peptiden und Lipiden) dieser genannten Stoffe.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge des einen oder der mehreren Antioxidantien in den erfindungsgemäßen Zusammensetzungen beträgt vorzugsweise von 0,001 bis 30,0 Gew.-%, besonders bevorzugt von 0,05 bis 20,0 Gew.-% und insbesondere bevorzugt von 1,0 bis 10,0 Gew.-%, bezogen auf das Gesamtgewicht der Zusammensetzung.

Des Weiteren können Feuchthaltemittel, ausgewählt aus dem Natriumsalz von 2-Pyrrolidone-5-carboxylat (NaPCA), Guanidin; Glycolsäure und deren Salzen, Milchsäure und deren Salzen, Glucosamine und deren Salzen, Lactamidmonoethanolamin, Acetamidmonoethanolamin, Harnstoff, Hydroxysäuren, Panthenol und dessen Derivaten, beispielsweise D-Panthenol (R-2,4-Dihydroxy-N-(3-hydroxypropyl)-3,3-dimethylbutamid), D,L-Panthenol, Calciumpantothenat, Panthetin, Pantothein, Panthenylethylether, Isopropylpalmitat, Glycerin und/oder Sorbitol eingesetzt werden, bevorzugt in Mengen von 0,1 bis 15,0 Gew.-% und besonders bevorzugt von 0,5 bis 5,0 Gew.-%, bezogen auf die fertigen Zusammensetzungen.

Zusätzlich können die erfindungsgemäßen Zusammensetzungen organische Lösungsmittel enthalten. Prinzipiell kommen als organische Lösungsmittel alle ein - oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45,0 Gew.-% und von Polyethylenglykol mit einer relativen Molekülmasse zwischen 400 und 600 in Mengen von 5,0 bis 25,0 Gew.-% bevorzugt. Weitere geeignete Lösungsmittel sind beispielsweise Triacetin (Glycerintriacetat) und 1-Methoxy-2-propanol.

Die erfindungsgemäßen Zusammensetzungen können ein oder mehrere Substanzen ausgewählt aus Farbmitteln, z.B. Farbstoffe und/oder Pigmente enthalten. Die in den erfindungsgemäßen Formulierungen enthaltenen Farbstoffe und/oder Pigmente, sowohl organische als auch anorganische Farbstoffe und Pigmente, sind aus der entsprechenden Positivliste der Kosmetikverordnung bzw. der EG-Liste kosmetischer Färbemittel ausgewählt.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphtalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-brezol-sulfosäure | 13015 | gelb |
| 2,4-Dihydroxy-azobenzol-4'-sulfosäure | 14270 | orange |
| 2-(2,4-Dimethylphenylazo-5-sulfosäurse)-1-hydroxynaphthalin-4-sulfosäure | 14700 | rot |
| 2-(4-Sulfo-1 -naphthylazo)-1 -naphthof-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8,-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-phrazolon-3-carbonsäure | 19140 | gelb |
| Pigment Yellow 16 | 20040 | gelb |
| 2,6-(4'-Sulfo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"Sulfo-1"-phenylazo)-7'-sulfo-1'-naphthylazo]-1-hydroxy-7-aminoaphthalin-3,6-disulfosäure | 27755 | schwarz |
| 4'-[(4"-Sulfo-1 "-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-ß-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino-phenyl-(2-sulfophenyl)-methylen-(N-ethyl-N-p-sulfo-benzyl)-cyclohexadienimin | 42090 | blau |
| Acid Green 9 | 42100 | grün |
| Diethyl-di-sulfobenzyl-di-4-amino-2-chlor-di-2-methyl-fuchsonimonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphtofuchsinimmonium | 44090 | grün |
| Acid red | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidine)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha, beta- bzw. gamma-Carotin | 75130 | orange |
| Keto- und/oder Hydroxylderivate des Carotins | 75135 | gelb |
| Guanin oder Periglanzmittel | 75170 | weiß |
| 1,7-Bis-(4-hydroxy-3-methoxyphenyl)1,6-heptadien-3,5-dion | 75300 | gelb |
| Komplexsalz (Na,Al,Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigmente Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydroxide | 77489 | orange |
| Eisenoxide und -hydroxide | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂*7H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyriliumsalze, Anthocyanine | | rot |
| Aluminium-, Zink-, Magnesium-, und Calciumstearat | | weiß |
| Bromthymolblau | | blau |
| Bromkresolgrün | | grün |
| Acid Red 195 | | rot |

Ferner vorteilhaft sind öllösliche Naturfarbstoffe, wie z. B. Paprikaextrakte, β-Carotin und Cochenille.

Vorteilhaft eingesetzt werden auch Perlglanzpigmente, z. B. Fischsilber (Guanin/Hypoxanthin-Mischkristalle aus Fischschuppen) und Perlmutt (vermahlene Muschelschalen), monokristalline Perlglanzpigmente wie z. B. Bismuthoxychlorid (BiOCI), Schicht-Substrat Pigmente, z. B. Glimmer/Metalloxid, silberweiße Perlglanzpigmente aus TiO₂, Interferenpigmente (TiO₂, unterschiedliche Schichtdicke), Farbglanzpigmente (Fe₂O₃) und Kombinationspigmente (TiO₂/Fe₂O₃, TiO₂/Cr₂O₃, TiO₂/Berliner Blau, TiO₂/Carmin).

Unter Effektpigmenten sind im Rahmen der vorliegenden Erfindung Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren. Als besondere Ausführungsform der Effektpigmente sind Interferenzpigmente bevorzugt. Besonders geeignete Effektpigmente sind beispielsweise Glimmerpartikel, die mit mindestens einem Metalloxid beschichtet sind. Neben Glimmer, einem Schichtsilikat, sind auch Kieselgel und andere SiO₂-Modifikationen als Träger geeignet. Ein häufig zur Beschichtung verwendetes Metalloxid ist beispielsweise Titanoxid, dem gewünschtenfalls Eisenoxid beigemischt sein kann. Über die Größe und die Form (z. B. sphärisch, ellipsoid, abgeflacht, eben, uneben) der Pigmentpartikel sowie über die Dicke der Oxidbeschichtung können die Reflexionseigenschaften beeinflusst werden. Auch andere Metalloxide, z. B. Bismutoxychlorid (BiOCl), sowie die Oxide von beispielsweise Titan, insbesondere die TiO₂-Modifikationen Anatas und Rutil, Aluminium, Tantal, Niob, Zirkon und Hafnium. Auch mit Magnesiumfluorid (MgF₂) und Calciumfluorid (Flussspat, CaF₂) können Effektpigmente hergestellt werden.

Die Effekte lassen sich sowohl über die Partikelgröße als auch über die Partikelgrößenverteilung des Pigmentensembles steuern. Geeignete Partikelgrößenverteilungen reichen z. B. von 2 - 50 µm, 5 - 25 µm, 5 - 40 µm, 5 - 60 µm, 5 - 95 µm, 5 - 100 µm, 10 - 60 µm, 10 - 100 µm, 10 - 125 µm, 20 - 100 µm, 20 - 150 µm, sowie < 15 µm. Eine breitere Teilchengrößenverteilung z. B. von 20 - 150 µm, ruft glitzernde Effekte hervor, während eine engere Teilchengrößenverteilung von < 15 µm für eine gleichmäßige seidige Erscheinung sorgt.

Die erfindungsgemäßen Zusammensetzungen enthalten Effektpigmente vorzugsweise in Mengen von 0,1 bis 20,0 Gew.%, besonders bevorzugt von 0,5 bis 10,0 Gew.-% und insbesondere bevorzugt von 1,0 bis 5,0 Gew.-%, jeweils bezogen auf das Gesamtgewicht der Zusammensetzungen.

Als periglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z. B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen.

Besonders bevorzugt sind Ethylenglykoldistearate und/oder Polyethylenglykoldistearate mit durchschnittlich 3 Glykoleinheiten.

Sofern die erfindungsgemäßen Zusammensetzungen periglanzgebende Verbindungen enthalten, sind diese bevorzugt in einer Menge von 0,1 bis 15,0 Gew.-% und besonders bevorzugt in einer Menge von 1,0 bis 10,0 Gew.-% in den erfindungsgemäßen Zusammensetzungen enthalten.

Als Duft- bzw. Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Trübungsmittel können Polymerdispersionen insbesondere Polyacrylatderivat-, Polyacrylamidderivat-, Poly(acrylatderivat-co-acrylamidderivat)-Dispersionen, Poly(styrolderivate-co-acrylatderivat)-Dispersionen, gesättigte und ungesätigte Fettalkohole verwendet werden.

An Silikonen können die zuvor unter den Silikonölen bzw. -wachsen genannten Substanzen verwendet werden.

Als Säuren oder Laugen zur pH-Wert Einstellung können vorzugsweise Mineralsäuren, insbesondere HCl, anorganische Basen, insbesondere NaOH oder KOH, und organische Säuren, insbesondere Zitronensäure, verwendet werden.

Die erfindungsgemäßen Zusammensetzungen haben pH-Werte von vorzugsweise 2 bis 10, besonders bevorzugt von 2 bis 7 und insbesondere bevorzugt von 2,5 bis 6,5.

In einer weiteren bevorzugten Ausführung der Erfindung liegen die erfindungsgemäßen Zusammensetzungen in der Form von Duschgelen oder Shampoos vor.

Die erfindungsgemäßen Polymere sind in vorteilhafter Weise als Verdicker, Konsistenzgeber, Emulgator, Sensorikadditiv, Solubilisator, Dispergator, Gleitmittel, Haftmittel, Stabilisator oder Fließgrenzenbildner geeignet.

Ein weiterer Gegenstand der Erfindung betrifft daher die Verwendung eines oder mehrerer der erfindungsgemäßen Polymere als Verdicker, Konsistenzgeber, Emulgator, Sensorikadditiv, Solubilisator, Dispergator, Gleitmittel, Haftmittel, Stabilisator oder Fließgrenzenbildner, vorzugsweise als Verdicker, Konsistenzgeber oder Fließgrenzenbildner, besonders bevorzugt als Verdicker oder Fließgrenzenbildner und insbesondere bevorzugt als Fließgrenzenbildner, außerordentlich bevorzugt in kosmetischen, dermatologischen oder pharmazeutischen Zusammensetzungen.

Die erfindungsgemäßen Polymere eignen sich besonders bevorzugt als als Verdicker, Konsistenzgeber, Emulgator, Sensorikadditiv, Solubilisator, Dispergator, Gleitmittel, Haftmittel, Stabilisator oder Fließgrenzenbildner, vorzugsweise als Verdicker, Konsistenzgeber oder Fließgrenzenbildner, besonders bevorzugt als Verdicker oder Fließgrenzenbildner und insbesondere bevorzugt als Fließgrenzenbildner, in flüssigen Zusammensetzungen, vorzugsweise in flüssigen kosmetischen, dermatologischen oder pharmazeutischen Zusammensetzungen, mit einem hohen Anteil an einem oder mehreren Tensiden.

Eine besonders bevorzugte Ausführungsform der Erfindung ist daher die Verwendung eines oder mehrerer der erfindungsgemäßen Polymere als Verdicker, Konsistenzgeber, Emulgator, Sensorikadditiv, Solubilisator, Dispergator, Gleitmittel, Haftmittel, Stabilisator oder Fließgrenzenbildner, vorzugsweise als Verdicker, Konsistenzgeber oder Fließgrenzenbildner, besonders bevorzugt als Verdicker oder Fließgrenzenbildner und insbesondere bevorzugt als Fließgrenzenbildner, in flüssigen Zusammensetzungen mit einem Tensidanteil von 0,01 bis 50,0 Gew.-%, vorzugsweise von 1,0 bis 30,0 Gew.-%, besonders bevorzugt von 5,0 bis 20,0 Gew.-% und insbesondere bevorzugt von 5,0 bis 15,0 Gew.-%, außerordentlich bevorzugt in kosmetischen, dermatologischen oder pharmazeutischen Zusammensetzungen.

In einer weiteren besonders bevorzugten Ausführungsform der Erfindung werden die erfindungsgemäßen Polymere als Verdicker oder Fließgrenzenbildner, vorzugsweise als Fließgrenzenbildner, zur Stabilisierung von anorganischen oder organischen Partikeln, von Öltröpfchen oder von Gasblasen verwendet, besonders bevorzugt in kosmetischen, dermatologischen oder pharmazeutischen Zusammensetzungen.

In einer weiteren besonders bevorzugten Ausführungsform der Erfindung werden die erfindungsgemäßen Polymere als Verdicker oder Fließgrenzenbildner, vorzugsweise als Fließgrenzenbildner, zur Stabilisierung von anorganischen oder organischen Partikeln, von Öltröpfchen oder von Gasblasen in Zusammensetzungen auf wässriger, wässrig- alkoholischer, wässriger tensidhaltiger oder wässrig-alkoholischer tensidhaltiger Basis verwendet, vorzugsweise in entsprechenden kosmetischen, dermatologischen oder pharmazeutischen Zusammensetzungen.

In einer weiteren besonders bevorzugten Ausführungsform der Erfindung werden die erfindungsgemäßen Polymere als Verdicker oder Fließgrenzenbildner, vorzugsweise als Fließgrenzenbildner, in flüssigen Zusammensetzungen enthaltend ein oder mehrere Tenside zur Stabilisierung von anorganischen oder organischen Partikeln, von Öltröpfchen oder von Gasblasen verwendet, vorzugsweise in flüssigen kosmetischen, dermatologischen oder pharmazeutischen Zusammensetzungen.

In einer insbesondere bevorzugten Ausführungsform der Erfindung werden die erfindungsgemäßen Polymere als Verdicker oder Fließgrenzenbildner, vorzugsweise als Fließgrenzenbildner, zur Stabilisierung von anorganischen oder organischen Partikeln, von Öltröpfchen oder von Gasblasen in Zusammensetzungen mit hohen Tensidkonzentrationen, vorzugsweise von 5,0 bis 20,0 Gew.-% und besonders bevorzugt von 5,0 bis 15,0 Gew.-%, verwendet. Bei den Zusammensetzungen handelt es sich vorzugsweise um kosmetische, dermatologische oder pharmazeutische Zusammensetzungen. Derartige Zusammensetzungen liegen vorzugsweise als klare Lösungen vor.

In einer weiteren bevorzugten Ausführungsform der Erfindung werden die erfindungsgemäßen Polymere zur Stabilisierung von Emulsionen, vorzugsweise von salzhaltigen Emulsionen, besonders bevorzugt von salzhaltigen kosmetischen, dermatologischen oder pharmazeutischen Emulsionen, verwendet.

Die erfindungsgemäßen Polymere sind säurestabil und eignen sich in vorteilhafter Weise zur Verwendung in kosmetischen, dermatologischen oder pharmazeutischen Zusammensetzungen mit niedrigem pH-Wert, insbesondere für die Pflege und Behandlung der Körper- oder Gesichtshaut.

Weiterer Gegenstand der vorliegenden Erfindung ist daher die Verwendung eines oder mehrerer erfindungsgemäßer Polymere zur Pflege und Behandlung der Körper- oder Gesichtshaut, vorzugsweise in kosmetischen, dermatologischen oder pharmazeutischen Zusammensetzungen, besonders bevorzugt in sauren kosmetischen, dermatologischen oder pharmazeutischen Zusammensetzungen.

Vorzugsweise finden die erfindungsgemäßen Verwendungen in kosmetischen, pharmazeutischen oder dermatologischen Zusammensetzungen statt.

Weiterhin vorteilhaft ist, dass die erfindungsgemäßen Polymere auch ohne Mitverwendung eines zusätzlichen Fließgrenzebildners und/oder ohne Mitverwendung eines zusätzlichen Verdickers in Zusammensetzungen, vorzugsweise in kosmetischen, dermatologischen oder pharmazeutischen Zusammensetzungen, eingesetzt werden können. Die Mitverwendung von zusätzlichen Fließgrenzebildnern und/oder Verdickern ist daher nicht zwingend, aber möglich. Eine Kombination mit anderen bekannten Fließgrenzenbildnern und/oder Verdickern kann zur Einstellung spezieller kosmetischer Profile und zur Ausnutzung synergistischer Effekte wünschenswert sein.

Weiterhin vorteilhaft ist, dass die erfindungsgemäßen Polymere auch ohne Mitverwendung eines zusätzlichen Fließgrenzebildners und/oder Verdickers in wässrigen, wässrig-alkoholischen, wässrigen tensidhaltigen, wässrig-alkoholischen tensidhaltigen Zusammensetzungen, vorzugsweise in entsprechenden kosmetischen, dermatologischen oder pharmazeutischen Zusammensetzungen, eingesetzt werden können. In einer insbesondere bevorzugten Ausführungsform der Erfindung liegen daher die erfindungsgemäßen Zusammensetzungen als wässrige tensidhaltige oder wässrig-alkoholische tensidhaltige Zusammensetzungen ohne Zusatz eines zusätzlichen Fließgrenzenbildners und/oder eines zusätzlichen Verdickers vor.

Die erfindungsgemäßen Polymere, die Struktureinheiten der Formel (2) enthalten, in denen n größer als 1 ist, zeichnen sich beispielsweise dadurch aus, dass sie beim Einsatz in entsprechenden Zusammensetzungen, insbesondere in entsprechenden kosmetischen, pharmazeutischen oder dermatologischen Zusammensetzungen, auch über einen weiten pH-Bereich und insbesondere bei pH-Werten von 2 bis 10 besonders vorteilhafte Fließgrenzen ausbilden und besonders klare Zusammensetzungen ergeben.

Die nachfolgenden Beispiele und Anwendungen sollen die Erfindung näher erläutern, ohne sie jedoch darauf zu beschränken.

### Beispiele:

Die nachfolgenden Beispiele und Anwendungen sollen die Erfindung näher erläutern, ohne sie jedoch darauf zu beschränken.

### 1. Polymerisation

### Allgemeine Polymerisationsvorschrift zur Herstellung der erfindungsgemäßen Polymere nach dem Fällungsverfahren in tert-Butanol

In einem 1-Liter Quickfitkolben mit Rückflusskühler, Gaseinleitung, Innenthermometer und Rührer werden 400 g tert.-Butanol vorgelegt und mit der berechneten Menge an 2-Acrylamido-2-methyl-1-propanesulfonsäure (AMPS^{®}, Lubrizol) versetzt. Anschließend wird durch NH₃-Einleitung neutralisiert (soll pH-Wert 6-7) und die berechnete Menge an 2-Carboxyethylacrylat (Sigma-Aldrich) oder 2-Carboxyethylacrylat Oligomerenmischung (Bimax Chemicals Ltd.) und die berechnete Vernetzermenge in die Reaktionsmischung hinzugegeben. Sollten weitere Comonomere benötigt werden, dann können diese nach der Neutralisation der Acrylamido-2-methyl-1-propanesulfonsulfonsäure (AMPS^{®}, Lubrizol) hinzugegeben werden. Sollte der pH-Wert der Reaktionsmischung nach der Comonomerenzugabe in den sauren Bereich gedriftet sein, so wird dieser durch NH₃-Einleitung, erneut neutralisiert (soll pH-Wert 6-7). Nach der Inertisierung der Mischung mit N₂ oder Argon wird bei einer Innentemperatur von 60 °C Dimethyl-2,2'-azobisisobutyrat (V-601) als Initiator zugesetzt und die Polymerisationsreaktion eingeleitet. Nach wenigen Minuten kommt es zum Ausfällen des fertigen Polymers. Die Mischung wird zwei Stunden auf Rückfluss erhitzt und das Polymerisat anschließend über eine Nutsche vom Lösemittel befreit und im Vakuum getrocknet. Diese Vorschrift ist allgemein auf alle im Weiteren beschriebenen Polymerisationsreaktionen anwendbar.

Die verwendete 2-Carboxyethylacrylat Oligomerenmischung von Bimax Chemicals Ltd. setzt sich aus 10 - 20 Gew.-% Acrylsäure, 30 - 60 Gew.-% 2-Carboxyethylacrylat und 30 - 60 Gew.-% höheren Oligomeren des 2-Carboxyethylacrylats zusammen.

### 2. Beispiel 1:

### Polymerisiert wurde nach der allgemeinen Polymerisationsvorschrift im Abschnitt 1).

| Reaktand | g | mol% |
|---|---|---|
| Acrylamido-2-methyl-1-propanesulfonsulfonsäure | 90 | 89,5 |
| 2-Carboxyethylacrylat Oligomermischung | 8,25 | 10,0 |
| Glycerol-propoxylate-triacrylate (GPTA) | 0,58 | 0,5 |
| Dimethyl-2,2'-azobisisobutyrat (V-601)* | 1,1 | - |

| | | |
|---|---|---|
| * bezogen auf die eingesetzte Monomerkonzentration | | |

### 3. Beispiel 2:

### Polymerisiert wurde nach der allgemeinen Polymerisationsvorschrift im Abschnitt 1).

| Reaktand | g | mol% |
|---|---|---|
| Acrylamido-2-methyl-1-propanesulfonsulfonsäure | 90 | 89,25 |
| 2-Carboxyethylacrylat Oligomermischung | 8,25 | 10,00 |
| Glycerol-propoxylate-triacrylate (GPTA) | 0,88 | 0,75 |
| Dimethyl-2,2'-azobisisobutyrat (V-601)* | 1,1 | - |

| | | |
|---|---|---|
| * bezogen auf die eingesetzte Monomerkonzentration | | |

### 4. Beispiel 3:

### Polymerisiert wurde nach der allgemeinen Polymerisationsvorschrift im Abschnitt 1).

| Reaktand | g | mol-% |
|---|---|---|
| Acrylamido-2-methyl-1-propanesulfonsulfonsäure | 90,0 | 87,5 |
| 2-Carboxyethylacrylat Oligomermischung | 8,4 | 12 |
| Pentaerythritol-diacrylat monostearat (PEAS) | 1,26 | 0,5 |
| Dimethyl-2,2'-azobisisobutyrat (V-601)* | 1,1 | - |

| | | |
|---|---|---|
| * bezogen auf die eingesetzte Monomerkonzentration | | |

### 5. Beispiel 4:

### Polymerisiert wurde nach der allgemeinen Polymerisationsvorschrift im Abschnitt 1).

| Reaktand | g | mol-% |
|---|---|---|
| Acrylamido-2-methyl-1-propanesulfonsulfonsäure | 90 | 85,5 |
| 2-Carboxyethylacrylat Oligomermischung | 8,6 | 10,0 |
| Dimethylacrylamid (DMAAm) | 2,0 | 4,0 |
| Pentaerythritol-diacrylat monostearat (PEAS) | 1,29 | 0,5 |
| Dimethyl-2,2'-azobisisobutyrat (V-601)* | 1,2 | - |

| | | |
|---|---|---|
| * bezogen auf die eingesetzte Monomerkonzentration | | |

### 6. Beispiel 5:

### Polymerisiert wurde nach der allgemeinen Polymerisationsvorschrift im Abschnitt 1).

| Reaktand | g | mol% |
|---|---|---|
| Acrylamido-2-methyl-1-propanesulfonsulfonsäure | 90 | 79,5 |
| 2-Carboxyethylacrylat Oligomermischung | 9,3 | 10,0 |
| Dimethylacrylamid (DMAAm) | 5,4 | 10,0 |
| Pentaerythritol-diacrylat monostearat (PEAS) | 1,36 | 0,5 |
| Dimethyl-2,2'-azobisisobutyrat (V-601)* | 1,3 | - |

| | | |
|---|---|---|
| * bezogen auf die eingesetzte Monomerkonzentration | | |

### 7. Beispiel 6:

### Polymerisiert wurde nach der allgemeinen Polymerisationsvorschrift im Abschnitt 1).

| Reaktand | g | mol-% |
|---|---|---|
| Acrylamido-2-methyl-1-propanesulfonsulfonsäure | 71,0 | 44,5 |
| 2-Carboxyethylacrylat | 17,0 | 15 |
| Dimethylacrylamid (DMAAm) | 30,5 | 40 |
| Trimethylolpropantriacrylat (TMPTA) | 1,2 | 0,5 |
| Dilaurylperoxid (DLP) | 1,7 | - |

| | | |
|---|---|---|
| * bezogen auf die eingesetzte Monomerkonzentration | | |

### 8. Beispiel 7:

### Polymerisiert wurde nach der allgemeinen Polymerisationsvorschrift im Abschnitt 1).

| Reaktand | g | mol-% |
|---|---|---|
| Acrylamido-2-methyl-1-propanesulfonsulfonsäure | 90 | 86,5 |
| 2-Carboxyethylacrylat | 10,25 | 12,0 |
| Dimethylacrylamid (DMAAm) | 0,5 | 1,0 |
| Diethylenglycoldimethacrylat (DEGDMA) | 0,61 | 0,5 |
| Dimethyl-2,2'-azobisisobutyrat (V-601)* | 1,20 | - |

| | | |
|---|---|---|
| * bezogen auf die eingesetzte Monomerkonzentration | | |

### 9. Beispiel 8:

### Polymerisiert wurde nach der allgemeinen Polymerisationsvorschrift im Abschnitt 1).

| Reaktand | g | mol% |
|---|---|---|
| Acrylamido-2-methyl-1-propanesulfonsulfonsäure | 90,0 | 88,0 |
| 2-Carboxyethylacrylat | 7,0 | 9,7 |
| n-Butylacrylat | 1,3 | 2,0 |
| Glycerol 1,3-diglycerolate diacrylate (GDDA) | 0,51 | 0,3 |
| Dimethyl-2,2'-azobisisobutyrat (V-601)* | 1,1 | - |

| | | |
|---|---|---|
| * bezogen auf die eingesetzte Monomerkonzentration | | |

### 10. Beispiel 9:

### Polymerisiert wurde nach der allgemeinen Polymerisationsvorschrift im Abschnitt 1).

| Reaktand | g | mol-% |
|---|---|---|
| Acrylamido-2-methyl-1-propanesulfonsulfonsäure | 90,0 | 86,8 |
| 2-Carboxyethylacrylat | 7,0 | 9,6 |
| Dimethylacrylamid (DMAAm) | 1,0 | 2,0 |
| 1,6-Hexandiol dimethacrylat (HDDMA) | 1,91 | 1,6 |
| Dimethyl-2,2'-azobisisobutyrat (V-601)* | 1,11 | - |

| | | |
|---|---|---|
| * bezogen auf die eingesetzte Monomerkonzentration | | |

### 11. Beispiel 10:

### Polymerisiert wurde nach der allgemeinen Polymerisationsvorschrift im Abschnitt 1).

| Reaktand | g | mol-% |
|---|---|---|
| Acrylamido-2-methyl-1-propanesulfonsulfonsäure | 90,0 | 87,7 |
| 2-Carboxyethylacrylat | 7,0 | 9,8 |
| Poly(ethylenglykol)monoacrylat | 3,8 | 2,0 |
| Glycerol-propoxylate-triacrylate (GPTA) | 0,59 | 0,5 |
| Dimethyl-2,2'-azobisisobutyrat (V-601)* | 1,10 | - |

| | | |
|---|---|---|
| * bezogen auf die eingesetzte Monomerkonzentration | | |

Die verwendete 2-Carboxyethylacrylat Oligomerenmischung von Bimax Chemicals Ltd. kann über chromatographische Methoden aufgereinigt werden. Beispielsweise kann die Acrylsäure abgetrennt werden und die so erhaltene Oligomerenmischung z. B. in den oben aufgeführten Beipielen 1 bis 5 eingesetzt werden.

### B) Beipiele zu kosmetischen Zusammensetzungen enthaltend erfindungsgemäße Polymere:

Bei allen folgenden Prozentangaben handelt es sich um Gewichtsprozent (Gew.-%), sofern nicht explizit anders angegeben.

Folgende kosmetische Formulierungen wurden mit erfindungsgemäßen Copolymeren hergestellt:

### Beispiel A: Anti-aging Cremegel

| | | |
|---|---|---|
| A | Genapol^{®} DAT | 2.00 % |
| | *PEG-150 Polyglyceryl-2 Tristearate and PEG-6 Caprylic*/*Capric Glyceride* | |
| | | |
| B | Wasser | ad 100 % |
| | | |
| C | Mineral Oil | 5.00 % |
| | SilCare^{®} Silicone 31 M50 | 3.00 % |
| | *Caprylyl Trimethicone* | |
| | | |
| D | Polymer nach Beispiel 4 | 1.80 % |
| | | |
| E | Glycolic Acid 30 % * | 6.00 % |
| | Phenonip^{®} | 0.50 % |
| | *Phenoxyethanol (and) Methylparaben (and) Ethylparaben (and) Butylparaben (and) Propylparaben (and) Isobutylparaben* | |
| | | |
| F | Genapol^{®} LA 070 | 3.00 % |
| | *Laureth-7* | |

### Herstellung

I Lösen von A in B unter Rühren und leichter Erwärmung
II D in C einrühren
III I zu II und Rühren bis ein homogenes Gel erhalten wird
IV E zu III hinzugeben
V F zu IV hinzugeben und verrühren

### Beispiel B: O/W Sonnenschutzcreme

| | | |
|---|---|---|
| A | Hostaphat^{®} CS 120 | 5.00 % |
| | *Stearyl Phosphate* | |
| | Tegin^{®} M | 2.50 % |
| | *Glyceryl Stearate* | |
| | Stearic Acid | 2.00 % |
| | Cetyl Alcohol | 1.00 % |
| | Abil^{®} 100 | 2.00 % |
| | *Dimethicone* | |
| | Mineral Oil, low viscosity | 3.00 % |
| | Cetiol^{®} 868 | 3.00 % |
| | *Ethylhexyl Stearate* | |
| | Velsan^{®} CCT | 3.00 % |
| | *Caprylic*/*Capric Triglyceride* | |
| | UV-Titan M210 | 5.00 % |
| | *Ultrafine Titanium Dioxide* | |
| | | |
| B | Polymer nach Beispiel 5 | 0.20 % |
| | | |
| C | Hostapon^{®} CLG | 0.60 % |
| | *Sodium Lauroyl Glutamate* | |
| | Eusolex^{®} 232 | 4.00 % |
| | *Phenylbenzimidazole Sulfonic Acid* | |
| | Tris (hydroxymethyl) aminomethan | q.s. |
| | *Tromethamine* | |
| | Allantoin | 0.20 % |
| | Glycerin | 5.00 % |
| | Konservierungsmittel | q.s |
| | Wasser | ad 100 % |
| | | |
| D | Parfum | 0.30 % |

### Herstellung

I A bei 80°C schmelzen, dann die Komponente B hinzugeben
II I homogenisieren
III C auf 80°C erwärmen
IV III in II einrühren
V IV bis zum Abkühlen rühren
VI D zu V bei 35°C hinzugeben
VII Emulsion homogenisieren

### Beispiel C: Shampoo mit Pearlizer

| | | |
|---|---|---|
| A | Wasser | ad 100 % |
| | | |
| B | Polymer nach Beispiel 2 | 2.50 % |
| | | |
| C | Genapol^{®} LRO liq. | 38.50 % |
| | *Sodium Laureth Sulfate* | |
| | Genagen^{®} CAB | 15.00 % |
| | *Cocamidopropyl Betaine* | |
| | Hostapon^{®} KCG | 8.30 % |
| | *Sodium Cocoyl Glutamate* | |
| | | |
| D | Genapol^{®} TSM | 4.00 % |
| | Na-Benzoat | 0.40 % |

### Herstellung

I B mit A mischen bis ein homogenes Gel erhalten wird
II Komponenten von C hinzugeben und bis zum kompletten Lösen rühren
III Komponenten von D zu II hinzugeben
IV pH-Wert auf pH = 4,5 einstellen

### Beispiel D: Sonnencreme mit Zinkoxid

| | | |
|---|---|---|
| A | Hostaphat^{®} KL 340 D | 1.00 % |
| | *Trilaureth-4 Phosphate* | |
| | Mineral Oil, low viscosity | 8.00 % |
| | *Isopropyl Palmitate* | |
| | Velsan^{®} CCT | 2.00 % |
| | *Caprylic*/*Capric Triglyceride* | |
| | Glyceryl Stearate | 0.50 % |
| | Cetearyl Alcohol | 0.50 % |
| | | |
| B | Polymer nach Beispiel 4 | 0.80 % |
| | | |
| C | Glycerin | 5.00 % |
| | Alkohol | 1.00 % |
| | Wasser | ad 100 % |
| | | |
| D | Tocopheryl Acetate | 1.00 % |
| | Z-Cote HP1 | 10.00 % |
| | *Zinc Oxide and Dimethicone* | |
| | Konservierungsmittel | q.s. |
| | | |
| E | Parfum | 0.30 % |

### Herstellung

I A bei 70°C schmelzen, dann B hinzufügen
II C auf 40°C erwärmen
III II in I einrühren
IV D bei 35°C in III einrühren
V E zu IV hinzugeben

### Beispiel E: Cremegel mit wasserlöslichem Sonnenschutz

| | | |
|---|---|---|
| A | Eusolex^{®} 232 | 2.00 % |
| | *Phenylbenzimidazole Sulfonic Acid* | |
| | | |
| B | Wasser | ad 100 % |
| | | |
| C | Polymer nach Beispiel 4 | 2.10 % |
| | | |
| D | Tegosoft TN | 5.00 % |
| | *C12-15 Alkylbenzoat* | |
| | SilCar^{®} Silicone 31 M50 | 3.00 % |
| | *Caprylyl Trimethicone* | |
| | | |
| E | Nipaguard^{®} MPA | q.s. |
| | *Benzyl Alcohol (and) Methylparaben (and) Propylparaben* | |
| | Genapol^{®} LA 070 | 2.50 % |
| | Laureth-7 | |
| | Parfum | q.s. |

### Herstellung

I A mit B mischen und auf etwa pH 7.3 neutralisieren
II C zugeben und Rühren bis ein homogenes Gel erhalten wird
III Komponenten von D mischen und zu II hinzugeben
IV E zu III hinzugeben

### Beispiel F: Whitening Gel

| | | |
|---|---|---|
| A | Genapol^{®} T 250 | 2.00 % |
| | *Ceteareth-25* | |
| | Genapol^{®} DAT 100 | 1.10 % |
| | *PEG-150 Polyglyceryl-2 Tristearate* | |
| | | |
| B | Wasser | ad 100.00 % |
| | | |
| C | Ascorbinsäure 2- Glucosid | 3.00 % |
| | Nipaguard^{®} DMDMH | q.s. |
| | *DMDM Hydantoin* | |
| | | |
| D | Polymer nach Beispiel 4 | 3.00 % |
| | | |
| E | Natriumhydroxid | q.s. |

### Herstellung

I Unter Rühren und leichtem Erwärmen Komponenten A in B lösen
II Bis zum Abkühlen (25°C) rühren und C zugeben
III D zugeben und Rühren bis ein homogenes Gel erhalten wird
IV Mit E einen pH-Wert von 6 einstellen

### Beispiel G: Anti-Schuppen-Shampoo

| | | |
|---|---|---|
| A | Wasser | ad 100 % |
| | | |
| B | Polymer nach Beispiel 1 | 2.50 % |
| | | |
| C | Genapol^{®} LRO liq. | 38.50 % |
| | *Sodium Laureth Sulfate* | |
| | Genagen^{®} CAB | 15.00 % |
| | *Cocamidopropyl Betaine* | |
| | Hostapon^{®} KCG | 8.30 % |
| | *Sodium Cocoyl Glutamate* | |
| | | |
| D | Zink Pyrithion | 1.80 % |
| | Konservierungsmittel | q.s. |

### Herstellung

I B mit A mischen und Rühren bis ein homogenes Gel erhalten wird
II Komponenten C zugeben und bis zum vollständigen Lösen rühren
III Komponenten von D zu II hinzugeben

### Beispiel H: Shower-Gel mit optischem Effekt (1)

| | | |
|---|---|---|
| A | Genapol^{®} LRO liq. | 49.95 % |
| | *Sodium Laureth Sulfate* | |
| | Genagen^{®} CAB | 5.00 % |
| | *Cocamidopropylbetaine* | |
| | | |
| B | Wasser | ad 100 % |
| | | |
| C | Polymer nach Beispiel 2 | 1.50 % |
| | | |
| D | Na-Sorbat | 0.40 % |
| | | |
| E | Citric acid | q.s. |
| | Cirebelle 104 | 0.20 % |

### Herstellung

I A mit B mischen
II Wenn gelöst, C zu I hinzugeben und Rühren bis eine homogene Lösung entsteht
III D zu II hinzugeben
IV pH-Wert mit E auf 5,0 einstellen

### Beispiel I: Shower-Gel mit optischem Effekt (2)

| | | |
|---|---|---|
| A | Genapol^{®} LRO liquid | 30.00% |
| | *Sodium Laureth Sulfate* | |
| | Hostapon^{®} KCG | 5.00 % |
| | *Sodium Cocoyl Glutamate* | |
| | Velsan^{®} CG 070 | 2.00% |
| | *PEG-7 Glyceryl Cocoate* | |
| | Genagen^{®} CAB | 6.00% |
| | *Cocamidopropyl Betaine* | |
| | Genapol^{®} LA 030 | 2.00% |
| | *Laureth-3* | |
| | | |
| B | Wasser | ad 100% |
| | | |
| C | Polymer nach Beispiel 3 | 1.50% |
| | | |
| D | Na-Sorbat | 0.30 % |

### Herstellung

I A mit B mischen bis die Komponenten vollständig gelöst sind
II C zu I hinzugeben; Rühren bis eine homogene Lösung entsteht.
III D zu II hinzugeben.

Die beim Rühren eingeschlossenen Luftblasen werden durch das erfindungsgemäße Polymer stabil dispergiert.

## Patentansprüche

1. Wasserlösliches oder wasserquellbares Polymer enthaltend
a) 20,0 bis 98,99 mol-% einer oder mehrerer voneinander unabhängiger wiederkehrender Struktureinheiten der Formel (1) 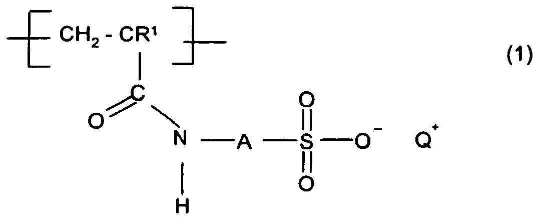 worin
R¹ Wasserstoff, Methyl oder Ethyl bedeutet,
A lineares oder verzweigtes C₁-C₁₂-Alkylen, vorzugsweise C₁-C₈-Alkylen, bedeutet, und
Q⁺ für H⁺, NH₄⁺, organische Ammoniumionen [HNR⁵R⁶R⁷]⁺, wobei R⁵, R⁶ und R⁷ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine C₆-C₂₂-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste R⁵, R⁶ und R⁷ nicht Wasserstoff ist, Li⁺, Na⁺, K⁺, ½ Ca⁺⁺, ½ Mg⁺⁺, ½ Zn⁺⁺ oder ⅓ Al⁺⁺⁺ oder für Mischungen aus diesen Ionen steht,
und
b) 1,0 bis 79,99 mol-% einer oder mehrerer voneinander unabhängiger wiederkehrender Struktureinheiten der Formel (2) 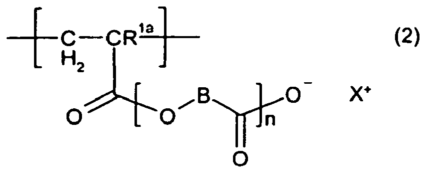 worin
R^{1a} Wasserstoff, Methyl oder Ethyl bedeutet,
X⁺ für H⁺, NH₄⁺, organische Ammoniumionen [HNR⁵R⁶R⁷]⁺, wobei R⁵, R⁶ und R⁷ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine C₆-C₂₂-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste R⁵, R⁶ und R⁷ nicht Wasserstoff ist, Li⁺, Na⁺, K⁺, ½ Ca⁺⁺, ½ Mg⁺⁺, ½ Zn⁺⁺ oder ⅓ Al⁺⁺⁺ oder für Mischungen aus diesen Ionen steht,
B eine lineare oder verzweigte Alkylengruppe mit 1 bis 6 Kohlenstoffatomen ist, und
n eine ganze Zahl von 1 bis 10 ist,
und
c) 0,01 bis 8,0 mol-%, vorzugsweise 0,01 bis 5,0 mol-%, besonders bevorzugt 0,01 bis 2,0 mol-% und insbesondere bevorzugt 0,25 bis 1,5 mol-% an einer oder mehreren voneinander unabhängigen wiederkehrenden vernetzenden Struktureinheiten, die aus einem oder mehreren Monomeren mit mindestens zwei olefinischen Doppelbindungen hervorgegangen sind.

2. Polymer nach Anspruch 1, **dadurch gekennzeichnet, dass** die eine oder die mehreren Struktureinheiten der Formel (1) abgeleitet sind von der 2-Acrylamido-2-methyl-propan-sulfonsäure.

3. Polymer nach Anspruch 1 oder 2, **dadurch gekennzeichnet, dass** der Neutralisationsgrad der einen oder der mehreren Struktureinheiten der Formel (1) von 50,0 bis 100 mol-%, besonders bevorzugt von 80,0 bis 100 mol-%, insbesondere bevorzugt von 90,0 bis 100 mol-% und außerordentlich bevorzugt von 95,0 bis 100 mol-% ist.

4. Polymer nach einem oder mehreren der Ansprüche 1 bis 3, **dadurch gekennzeichnet, dass** der Anteil an der einen oder den mehreren Struktureinheiten der Formel (2), worin n eine ganze Zahl von 2 oder mehr ist, innerhalb der Komponente b) der erfindungsgemäßen Polymere mindestens 10,0 mol-%, besonders bevorzugt mindestens 20,0 mol-%, insbesondere bevorzugt mindestens 25,0 mol-%, außerordentlich bevorzugt mindestens 30,0 mol-% ist.

5. Polymer nach einem oder mehreren der Ansprüche 1 bis 4, **dadurch gekennzeichnet, dass** in der einen oder den mehreren Struktureinheiten der Formel (2) B die Gruppe -CH₂CH₂- und n eine ganze Zahl von 1 bis 5 und vorzugsweise von 1 bis 4 ist.

6. Polymer nach einem oder mehreren der Ansprüche 1 bis 5, **dadurch gekennzeichnet, dass** das von H⁺ verschiedene Gegenion Q⁺ in der einen oder den mehreren Struktureinheiten der Formel (1) ausgewählt ist aus NH₄⁺, Alkali⁺, wobei unter Alkali⁺ wiederum Na⁺ bevorzugt ist, Erdalkali⁺⁺ und Mischungen aus diesen Ionen und besonders bevorzugt NH₄⁺ ist und das Gegenion X⁺ in der einen oder den mehreren Struktureinheiten der Formel (2) ausgewählt ist aus H⁺, NH₄⁺, Alkali⁺, wobei unter Alkali⁺ wiederum Na⁺ bevorzugt ist, Erdalkali⁺⁺ und Mischungen aus diesen Ionen und besonders bevorzugt H⁺ und/oder NH₄⁺ ist.

7. Polymer nach einem oder mehreren der Ansprüche 1 bis 6, **dadurch gekennzeichnet, dass** es
a) 50,0 bis 96,99 mol-%, vorzugsweise 75,0 bis 94,99 mol-% und besonders bevorzugt 84,75 bis 94,75 mol-% an der einen oder den mehreren der Struktureinheiten der Formel (1),
b) 3,0 bis 49,99 mol-%, vorzugsweise 5,0 bis 24,99 mol-% und besonders bevorzugt 5,0 bis 15,0 mol% an der einen oder den mehreren der Struktureinheiten der Formel (2), und
c) 0,01 bis 5,0 mol-%, vorzugsweise 0,01 bis 2,0 mol-% und besonders bevorzugt 0,25 bis 1,5 mol-% an der einen oder den mehreren vernetzenden Struktureinheiten der Komponente c) enthält.

8. Polymer nach einem oder mehreren der Ansprüche 1 bis 7, **dadurch gekennzeichnet, dass** es eine oder mehrere voneinander unabhängige wiederkehrende vernetzende Struktureinheiten der Formel (3) 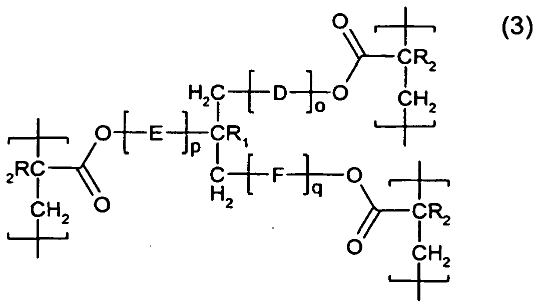 gemäß Komponente c) enthält, worin
R¹ und R² jeweils unabhängig voneinander Wasserstoff, Methyl oder Ethyl bedeuten,
D, E und F jeweils unabhängig voneinander Methylenoxy (-CH₂-O-), Ethylenoxy (-CH₂-CH₂-O-), Popylenoxy (-CH(CH₃)-CH₂-O-), eine lineare oder verzweigte Alkylengruppe mit 1 bis 6 Kohlenstoffatomen, eine lineare oder verzweigte, einfach oder mehrfach ungesättigte Alkenylengruppe mit 2 bis 6 Kohlenstoffatomen, eine lineare Mono-Hydroxyalkylengruppe mit 2 bis 6 Kohlenstoffatomen oder eine lineare oder verzweigte Di-Hydroxyalkylengruppe mit 3 bis 6 Kohlenstoffatomen bedeuten, und
o, p und q jeweils unabhängig voneinander eine ganze Zahl von 1 bis 50
bedeuten.

9. Polymer nach einem oder mehreren der Ansprüche 1 bis 8, **dadurch gekennzeichnet, dass** es
ab) 29,99 bis 98,99 mol-%, vorzugsweise 62,0 bis 98,99 mol-%, einer Mischung der wiederkehrenden Struktureinheiten der Formeln (1) und (2),
c) 0,01 bis 8,0 mol-% an der einen oder den mehreren vernetzenden Struktureinheiten der Komponente c), und
d) 0,01 bis 70,0 mol-%, vorzugsweise 1,0 bis 70,0 mol-%, besonders bevorzugt 1,0 bis 37,99 mol-%, einer oder mehrerer voneinander unabhängiger wiederkehrender nicht-vernetzender Struktureinheiten, die hervorgegangen sind aus einer oder mehreren Verbindungen der allgemeinen Formel (4) 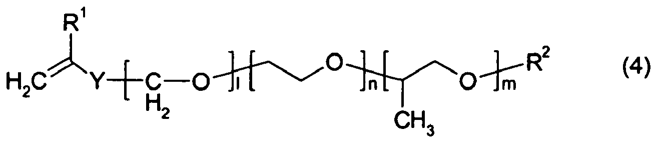 worin
R¹ Wasserstoff, Methyl oder Ethyl bedeutet,
R² H, eine lineare oder verzweigte Alkylgruppe mit 1 bis 50 Kohlenstoffatomen, eine lineare oder verzweigte Mono-Hydroxyalkylgruppe mit 2 bis 6 Kohlenstoffatomen, eine lineare oder verzweigte Di-Hydroxyalkylgruppe mit 2 bis 6 Kohlenstoffatomen, -(CO-O-R⁷-)ₒR⁸ oder -(CO-NR⁵-R⁷-)ₚR⁸ bedeutet,
l, m, n, o und p jeweils unabhängig voneinander eine ganze Zahl von 0 bis 300 bedeuten,
Y eine chemische Bindung, O, NR³, S, PR³, CH₂, CH₂O, CH₂NR³, CH₂S, C(O), C(NR³), C(O)O, OC(O), C(O)NR³, NR³C(O), C(NR⁴)NR³, C(O)S, R⁶OC(O)O, R⁶OC(O)NR³, R⁶R³NC(O)NR⁴, R⁶NR³C(NR⁵)NR⁴, R⁶OC(O)S, R⁶P(O)O, R⁶OP(O)O, R⁶S(O), R⁶S(O)(O), R⁶S(O)O, R⁶S(O)(O)O, R⁶OS(O)O oder R⁶OS(O)(O)O, vorzugsweise eine chemische Bindung, O, CH₂, C(O)O, OC(O), C(O)NR³ oder NR³C(O) bedeutet,
R³, R⁴, R⁵ und R⁸ jeweils unabhängig voneinander Wasserstoff oder einen linearen oder verzweigten Alkylrest mit 1 bis 50 C-Atomen bedeuten,
R⁶ eine chemische Bindung oder CH₂ bedeutet und
R⁷ einen linearen oder verzweigten Alkylenrest mit 1 bis 50 C-Atomen bedeutet.

10. Polymer nach Anspruch 9, **dadurch gekennzeichnet, dass** die eine oder die mehreren Struktureinheiten der Komponente d) ausgewählt sind aus einer oder mehreren Struktureinheiten der Formel (5) 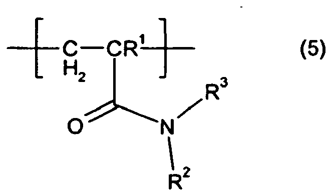 worin
R¹ Wasserstoff, Methyl oder Ethyl bedeutet und
R² und R³ jeweils unabhängig voneinander Wasserstoff, Methyl, Ethyl, n-Propyl oder iso-Propyl bedeuten, und mindestens einer der Reste R² und R³ nicht Wasserstoff ist, und
einer oder mehreren der Struktureinheiten der Formel (6) 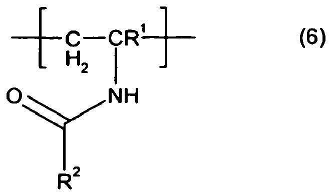 worin
R¹ Wasserstoff, Methyl oder Ethyl bedeutet und
R² Wasserstoff, Methyl, Ethyl, n-Propyl oder iso-Propyl ist.

11. Verfahren zur Herstellung eines Polymers nach einem oder mehreren der Ansprüche 1 bis 10, **dadurch gekennzeichnet, dass** Monomere, aus denen sich die Struktureinheiten der Komponenten a) bis c) und gegebenenfalls d) ableiten sowie gegebenenfalls weitere Monomere, in einem protischen Lösungsmittel, vorzugsweise in tert.-Butanol, radikalisch polymerisiert werden, und gegebenenfalls die Monomere vor der Polymerisation oder das Polymer nach der Polymerisation mit Ammoniak oder organischen Aminen oder einer Li⁺-, Na⁺-, K⁺-, Ca⁺⁺-, Mg⁺⁺-, Zn⁺⁺- oder Al⁺⁺⁺-enthaltenden Base, vorzugsweise mit den entsprechenden Hydroxiden oder Carbonaten und besonders bevorzugt mit Hydroxiden, neutralisiert werden.

12. Kosmetische, dermatologische oder pharmazeutische Zusammensetzung enthaltend ein oder mehrere Polymere nach einem oder mehreren der Ansprüche 1 bis 10, vorzugsweise in einer Menge von 0,01 bis 10,0 Gew.-%, besonders bevorzugt in einer Menge von 0,1 bis 5,0 Gew.-% und insbesondere bevorzugt in einer Menge von 0,5 bis 2,0 Gew.-%, bezogen auf die fertige Zusammensetzung.

13. Zusammensetzung nach Anspruch 12, **dadurch gekennzeichnet, dass** sie in Form eines Fluids, Gels, Öls, Schaums, Sprays, einer Lotion oder Creme vorliegt.

14. Zusammensetzung nach Anspruch 12 oder 13, **dadurch gekennzeichnet, dass** sie ein oder mehrere Tenside enthält, vorzugsweise in einer Menge von 0,01 bis 50,0 Gew.-%, besonders bevorzugt von 1,0 bis 30,0 Gew.%, insbesondere bevorzugt von 5,0 bis 20,0 Gew.-% und außerordendlich bevorzugt von 5,0 bis 15,0 Gew.-%, bezogen auf die fertige Zusammensetzung.

15. Zusammensetzung nach einem oder mehreren der Ansprüche 12 bis 14, **dadurch gekennzeichnet, dass** sie ein oder mehrere Substanzen ausgewählt aus anorganischen und organischen Salzen enthält.

16. Zusammensetzung nach einem oder mehreren der Ansprüche 12 bis 15, **dadurch gekennzeichnet, dass** sie ein oder mehrere Substanzen ausgewählt aus alpha- und beta-Hydroxysäuren enthält.

17. Zusammensetzung nach einem oder mehreren der Ansprüche 12 bis 16, **dadurch gekennzeichnet, dass** sie ein oder mehrere Substanzen ausgewählt aus Vitamin C und Vitamin C-Derivaten enthält, wobei die Vitamin C-Derivate vorzugsweise ausgewählt sind aus Natriumascorbylphosphat, Magnesiumascorbylphoshat und Magnesiumascorbylglucosid.

18. Zusammensetzung nach einem oder mehreren der Ansprüche 12 bis 17, **dadurch gekennzeichnet, dass** sie eine oder mehrere Substanzen ausgewählt aus Benzoesäure, Sorbinsäure, Salicylsäure, Milchsäure und Paramethoxybenzoesäure enthält.

19. Zusammensetzung nach einem oder mehreren der Ansprüche 12 bis 18, **dadurch gekennzeichnet, dass** sie ein oder mehrere Substanzen ausgewählt aus anorganischen und organischen UV-Filtern enthält und vorzugsweise in der Form einer Sonnenschutzzusammensetzung vorliegt.

20. Zusammensetzung nach einem oder mehreren der Ansprüche 12 bis 19, **dadurch gekennzeichnet, dass** sie einen pH-Wert von 2 bis 10, vorzugsweise von 2 bis 7 und besonders bevorzugt von 2,5 bis 6,5 besitzt.

21. Zusammensetzung nach einem oder mehreren der Ansprüche 12 bis 20, **dadurch gekennzeichnet, dass** sie in der Form eines Duschgels oder Shampoos vorliegt.

22. Verwendung eines oder mehrerer der Polymere nach einem oder mehreren der Ansprüche 1 bis 10 als Verdicker, Konsistenzgeber, Emulgator, Sensorikadditiv, Solubilisator, Dispergator, Gleitmittel, Haftmittel, Stabilisator oder Fließgrenzenbildner, vorzugsweise als Verdicker, Konsistenzgeber oder Fließgrenzenbildner, besonders bevorzugt als Verdicker oder Fließgrenzenbildner und insbesondere bevorzugt als Fließgrenzenbildner, außerordentlich bevorzugt in kosmetischen, dermatologischen oder pharmazeutischen Zusammensetzungen.

23. Verwendung nach Anspruch 22 als Verdicker oder Fließgrenzenbildner, vorzugsweise als Fließgrenzenbildner, zur Stabilisierung von anorganischen oder organischen Partikeln, von Öltröpfchen oder von Gasblasen, besonders bevorzugt in kosmetischen, dermatologischen oder pharmazeutischen Zusammensetzungen.

24. Verwendung nach Anspruch 22 oder 23 als Verdicker oder Fließgrenzenbildner, vorzugsweise als Fließgrenzenbildner, in flüssigen Zusammensetzungen enthaltend ein oder mehrere Tenside zur Stabilisierung von anorganischen oder organischen Partikeln, von Öltröpfchen oder von Gasblasen, vorzugsweise in flüssigen kosmetischen, dermatologischen oder pharmazeutischen Zusammensetzungen.

25. Verwendung nach Anspruch 22 zur Stabilisierung von Emulsionen, vorzugsweise von salzhaltigen Emulsionen, besonders bevorzugt von salzhaltigen kosmetischen, dermatologischen oder pharmazeutischen Emusionen.

## Claims

1. A water-soluble or water-swellable polymer comprising
a) 20.0 to 98.99 mol% of one or more independent repeating structural units of the formula (1) 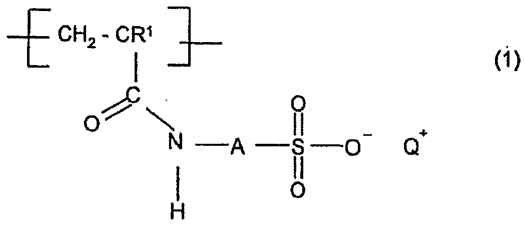 in which
R¹ is hydrogen, methyl or ethyl,
A is linear or branched C₁-C₁₂ alkylene, preferably C₁-C₈ alkylene, and
Q⁺ is H⁺, NH₄⁺, organic ammonium ions [NHR⁵R⁶R⁷]⁺ where R⁵, R⁶, and R⁷ independently of one another may be hydrogen, a linear or branched alkyl group having 1 to 22 carbon atoms, a linear or branched, singularly or multiply unsaturated alkenyl group having 2 to 22 carbon atoms, a C₆-C₂₂ alkylamidopropyl group, a linear monohydroxyalkyl group having 2 to 10 carbon atoms or a linear or branched dihydroxyalkyl group having 3 to 10 carbon atoms, and where at least one of the radicals R⁵, R⁶, and R⁷ is not hydrogen, or Q⁺ is Li⁺, Na⁺, K⁺, ½ Ca⁺⁺, ½ Mg⁺⁺, ½ Zn⁺⁺ or ⅓ Al⁺⁺⁺, or is mixtures of these ions,
and
b) 1.0 to 79.99 mol% of one or more independent repeating structural units of the formula (2) 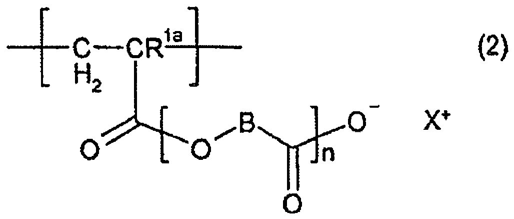 in which
R^{1a} is hydrogen, methyl or ethyl,
X⁺ is H⁺, NH₄⁺, organic ammonium ions [NHR⁵R⁶R⁷]⁺ where R⁵, R⁶, and R⁷ independently of one another may be hydrogen, a linear or branched alkyl group having 1 to 22 carbon atoms, a linear or branched, singularly or multiply unsaturated alkenyl group having 2 to 22 carbon atoms, a C₆-C₂₂ alkylamidopropyl group, a linear monohydroxyalkyl group having 2 to 10 carbon atoms or a linear or branched dihydroxyalkyl group having 3 to 10 carbon atoms, and where at least one of the radicals R⁵, R⁶, and R⁷ is not hydrogen, or X⁺ is Li⁺, Na⁺, K⁺, ½ Ca⁺⁺, ½ Mg⁺⁺, ½ Zn⁺⁺ or ⅓ Al⁺⁺⁺, or is mixtures of these ions,
B is a linear or branched alkylene group having 1 to 6 carbon atoms, and
n is an integer from 1 to 10,
and
c) 0.01 to 8.0 mol%, preferably 0.01 to 5.0 mol%, more preferably 0.01 to 2.0 mol%, and with particular preference 0.25 to 1.5 mol% of one or more independent repeating crosslinking structural units, originating from one or more monomers having at least two olefinic double bonds.

2. A polymer as claimed in claim 1, **characterized in that** the one or more structural units of the formula (1) derive from 2-acrylamido-2-methylpropanesulfonic acid.

3. A polymer as claimed in claim 1 or 2, **characterized in that** the degree of neutralization of the one or more structural units of the formula (1) is from 50.0 to 100 mol%, more preferably from 80.0 to 100 mol%, with particular preference from 90.0 to 100 mol%, and very preferably from 95.0 to 100 mol%.

4. A polymer as claimed in one or more of claims 1 to 3, **characterized in that** the fraction of the one or more structural units of the formula (2) in which n is an integer of two or more within component b) of the polymers of the invention is at least 10.0 mol%, more preferably at least 20.0 mol%, with particular preference at least 25.0 mol%, very preferably at least 30.0 mol%.

5. A polymer as claimed in one or more of claims 1 to 4, **characterized in that**, in the one or more structural units of the formula (2), B is the group -CH₂CH₂- and n is an integer from 1 to 5 and preferably from 1 to 4.

6. A polymer as claimed in one or more of claims 1 to 5, **characterized in that** the non-H⁺ counterion Q⁺ in the one or more structural units of the formula (1) is selected from NH₄⁺, alkali⁺, where alkali⁺ in turn is preferably Na⁺, alkaline earth⁺⁺, and mixtures of these ions, and more preferably is NH₄⁺, and the counterion X⁺ in the one or more structural units in the formula (2) is selected from H⁺, NH₄⁺, alkali⁺, where alkali⁺ in turn is preferably Na⁺, alkaline earth⁺⁺, and mixtures of these ions, and more preferably is H⁺ and/or NH₄⁺.

7. A polymer as claimed in one or more of claims 1 to 6, **characterized in that** it comprises
a) 50.0 to 96.99 mol%, preferably 75.0 to 94.99 mol%, and more preferably 84.75 to 94.75 mol% of the one or more structural units of the formula (1),
b) 3.0 to 49.99 mol%, preferably 5.0 to 24.99 mol%, and more preferably 5.0 to 15.0 mol% of the one or more structural units of the formula (2), and
c) 0.01 to 5.0 mol%, preferably 0.01 to 2.0 mol%, and more preferably 0.25 to 1.5 mol% of the one or more crosslinking structural units of component c).

8. A polymer as claimed in one or more of claims 1 to 7, **characterized in that** it comprises one or more independent repeating crosslinking structural units of the formula (3) 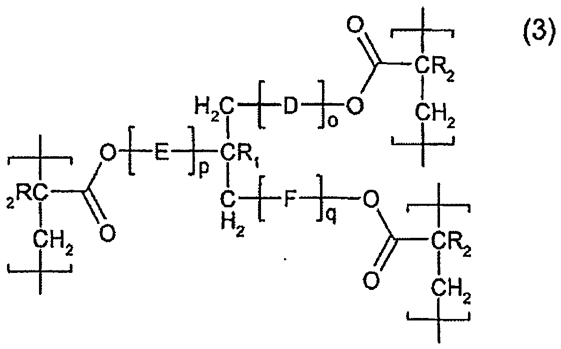 according to component c), in which
R¹ and R² each independently are hydrogen, methyl or ethyl,
D, E, and F each independently are methyleneoxy (-CH₂-O-), ethyleneoxy (-CH₂-CH₂-O-), propyleneoxy (-CH(CH₃)-CH₂-O-), a linear or branched alkylene group having 1 to 6 carbon atoms, a linear or branched, singularly or multiply unsaturated alkenylene group having 2 to 6 carbon atoms, a linear mono-hydroxyalkylene group having 2 to 6 carbon atoms or a linear or branched dihydroxyalkylene group having 3 to 6 carbon atoms, and
o, p, and q each independently are an integer from 1 to 50.

9. A polymer as claimed in one or more of claims 1 to 8, **characterized in that** it comprises
ab) 29.99 to 98.99 mol%, preferably 62.0 to 98.99 mol%, of a mixture of the repeating structural units of the formulae (1) and (2),
c) 0.01 to 8.0 mol% of the one or more crosslinking structural units of component c), and
d) 0.01 to 70.0 mol%, preferably 1.0 to 70.0 mol%, more preferably 1.0 to 37.99 mol% of one or more independent repeating noncrosslinking structural units, originating from one or more compounds of the formula (4) 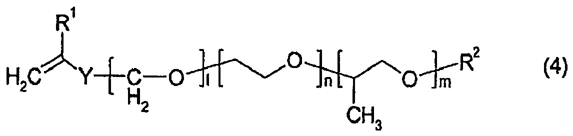 in which
R¹ is hydrogen, methyl or ethyl,
R² is H, a linear or branched alkyl group having 1 to 50 carbon atoms, a linear or branched monohydroxyalkyl group having 2 to 6 carbon atoms, a linear or branched dihydroxyalkyl group having 2 to 6 carbon atoms, (-CO-O-R⁷-)ₒR⁸ or -(CO-NR⁵-R⁷) ₚR⁸,
l, m, n, o, and p each independently are an integer from 0 to 300,
Y is a chemical bond, O, NR³, S, PR³, CH₂, CH₂O, CH₂NR³, CH₂S, C(O), C(NR³), C(O)O, OC(O), C(O)NR³, NR³C(O), C(NR⁴)NR³, C(O)S, R⁶OC(O)O, R⁶OC(O)NR³, R⁶R³NC(O)NR⁴, R⁶NR³C(NR⁵)NR⁴, R⁶OC(O)S, R⁶P(O)O, R⁶OP(O)O, R⁶S(O), R⁶S(O)(O), R⁶S(O)O, R⁶S(O)(O)O, R⁶OS(O)O or R⁶OS(O)(O)O, preferably a chemical bond, O, CH₂, C(O)O, OC(O), C(O)NR³ or NR³C(O),
R³, R⁴, R⁵, and R⁸ each independently are hydrogen or a linear or branched alkyl radical having 1 to 50 C atoms,
R⁶ is a chemical bond or CH₂, and
R⁷ is a linear or branched alkylene radical having 1 to 50 C atoms.

10. A polymer as claimed in claim 9, **characterized in that** the one or more structural units of component d) are selected from one or more structural units of the formula (5) 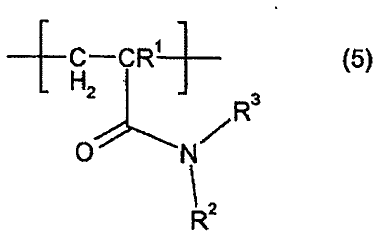 in which
R¹ is hydrogen, methyl or ethyl and
R² and R³ each independently are hydrogen, methyl, ethyl, n-propyl or isopropyl, and at least one of the radicals R² and R³ is not hydrogen, and
one or more of the structural units of the formula (6) 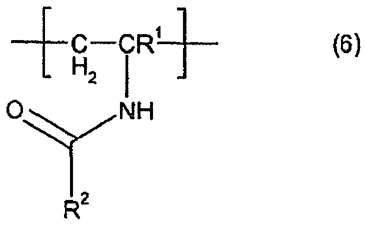 in which
R¹ is hydrogen, methyl or ethyl and
R² is hydrogen, methyl, ethyl, n-propyl or isopropyl.

11. A process for preparing a polymer as claimed in one or more of claims 1 to 10, **characterized in that** monomers from which the structural units of components a) to c) and optionally d) derive and also, optionally, further monomers are radically polymerized in a protic solvent, preferably in tert-butanol, and optionally the monomers before the polymerization or the polymer after the polymerization are or is neutralized with ammonia or organic amines or with a base containing Li⁺, Na⁺, K⁺, Ca⁺⁺, Mg⁺⁺, Zn⁺⁺ or Al⁺⁺⁺, preferably with the corresponding hydroxides or carbonates, and more preferably with hydroxides.

12. A cosmetic, dermatological or pharmaceutical composition comprising one or more polymers as claimed in one or more of claims 1 to 10, preferably in an amount of 0.01% to 10.0% by weight, more preferably in an amount of 0.1% to 5.0% by weight, and with particular preference in an amount of 0.5% to 2.0% by weight, based on the completed composition.

13. A composition as claimed in claim 12, **characterized in that** it is in the form of a fluid, gel, oil, foam, spray, lotion or cream.

14. A composition as claimed in claim 12 or 13, **characterized in that** it comprises one or more surfactants, preferably in an amount of 0.01% to 50.0% by weight, more preferably of 1.0% to 30.0% by weight, with particular preference of 5.0% to 20.0% by weight, and very preferably of 5.0% to 15.0% by weight, based on the completed composition.

15. A composition as claimed in one or more of claims 12 to 14, **characterized in that** it comprises one or more substances selected from organic and inorganic salts.

16. A composition as claimed in one or more of claims 12 to 15, **characterized in that** it comprises one or more substances selected from alpha- and beta-hydroxy acids.

17. A composition as claimed in one or more of claims 12 to 16, **characterized in that** it comprises one or more substances selected from vitamin C and vitamin C derivatives, the vitamin C derivatives being preferably selected from sodium ascorbyl phosphate, magnesium ascorbyl phosphate, and magnesium ascorbyl glucoside.

18. A composition as claimed in one or more of claims 12 to 17, **characterized in that** it comprises one or more substances selected from benzoic acid, sorbic acid, salicylic acid, lactic acid, and para-methoxybenzoic acid.

19. A composition as claimed in one or more of claims 12 to 18, **characterized in that** it comprises one or more substances selected from organic and inorganic UV filters and is present preferably in the form of a sun protection composition.

20. A composition as claimed in one or more of claims 12 to 19, **characterized in that** it possesses a pH of 2 to 10, preferably of 2 to 7, and more preferably of 2.5 to 6.5.

21. A composition as claimed in one or more of claims 12 to 20, **characterized in that** it is in the form of a shower gel or shampoo.

22. The use of one or more of the polymers as claimed in one or more of claims 1 to 10 as thickener, consistency modifier, emulsifier, sensorial additive, solubilizer, dispersant, lubricant, adherent, stabilizer or yield point former, preferably as thickener, consistency modifier or yield point former, more preferably as thickener or yield point former, and with more particular preference as yield point former, very preferably in cosmetic, dermatological or pharmaceutical compositions.

23. The use as claimed in claim 22 as thickener or yield point former, preferably as yield point former, for stabilizing organic or inorganic particles, oil droplets or gas bubbles, more preferably in cosmetic, dermatological or pharmaceutical compositions.

24. The use as claimed in claim 22 or 23 as thickener or yield point former, preferably as yield point former, in liquid compositions comprising one or more surfactants for stabilizing organic or inorganic particles, oil droplets or gas bubbles, preferably in liquid cosmetic, dermatological or pharmaceutical compositions.

25. The use as claimed in claim 22 for stabilizing emulsions, preferably salt-containing emulsions, more preferably salt-containing cosmetic, dermatological or pharmaceutical emulsions.

## Revendications

1. Polymère soluble dans l'eau ou gonflable dans l'eau, contenant
a) 20,0 à 98,99 % en moles d'une ou de plusieurs unités structurales de répétition de formule (1) indépendantes les unes des autres 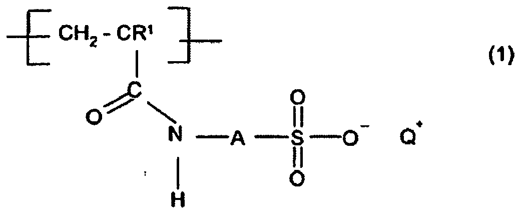 dans laquelle
R¹ signifie hydrogène, méthyle ou éthyle,
A signifie alkylène en C₁-C₁₂ linéaire ou ramifié, de préférence alkylène en C₁-C₈, et
Q⁺ signifie H⁺, NH₄⁺, des ions ammonium organiques [HNR⁵R⁶R⁷]⁺, R⁵, R⁶ et R⁷ pouvant être indépendamment les uns des autres l'hydrogène, un groupe alkyle linéaire ou ramifié de 1 à 22 atomes de carbone, un groupe alcényle linéaire ou ramifié, une ou plusieurs fois insaturé, de 2 à 22 atomes de carbone, un groupe alkylamidopropyle en C₆-C₂₂, un groupe monohydroxyalkyle linéaire de 2 à 10 atomes de carbone ou un groupe dihydroxyalkyle linéaire ou ramifié de 3 à 10 atomes de carbone, et au moins un des radicaux R⁵, R⁶ et R⁷ n'étant pas l'hydrogène, Li⁺, Na⁺, K⁺, ½ Ca⁺⁺, ½ Mg⁺⁺, ½ Zn⁺⁺ ou ⅓ Al⁺⁺⁺, ou des mélanges de ces ions,
et
b) 1,0 à 79,99 % en moles d'une ou de plusieurs unités structurales de répétition de formule (2) indépendantes les unes des autres 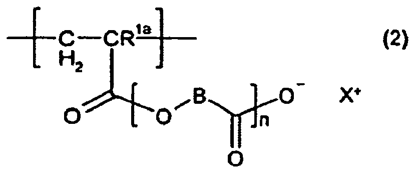 dans laquelle
R^{1a} signifie hydrogène, méthyle ou éthyle,
X⁺ signifie H⁺, NH₄⁺, des ions ammonium organiques [HNR⁵R⁶R⁷]⁺, R⁵, R⁶ et R⁷ pouvant être indépendamment les uns des autres l'hydrogène, un groupe alkyle linéaire ou ramifié de 1 à 22 atomes de carbone, un groupe alcényle linéaire ou ramifié, une ou plusieurs fois insaturé, de 2 à 22 atomes de carbone, un groupe alkylamidopropyle en C₆-C₂₂, un groupe monohydroxyalkyle linéaire de 2 à 10 atomes de carbone ou un groupe dihydroxyalkyle linéaire ou ramifié de 3 à 10 atomes de carbone, et au moins un des radicaux R⁵, R⁶ et R⁷ n'étant pas l'hydrogène, Li⁺, Na⁺, K⁺, ½ Ca⁺⁺, ½ Mg⁺⁺, ½ Zn⁺⁺ ou ⅓ Al⁺⁺⁺, ou des mélanges de ces ions,
B est un groupe alkylène linéaire ou ramifié de 1 à 6 atomes de carbone, et
n est un nombre entier de 1 à 10,
et
c) 0,01 à 8,0 % en moles, de préférence 0,01 à 5,0 % en moles, de manière particulièrement préférée 0,01 à 2,0 % en moles et de manière notamment préférée 0,25 à 1,5 % en moles d'une ou de plusieurs unités structurales de répétition réticulantes indépendantes les unes des autres, qui proviennent d'un ou de plusieurs monomères contenant au moins deux doubles liaisons oléfiniques.

2. Polymère selon la revendication 1, **caractérisé en ce que** la ou les unités structurales de formule (1) sont dérivées d'acide 2-acrylamido-2-méthylpropane-sulfonique.

3. Polymère selon la revendication 1 ou 2, **caractérisé en ce que** le degré de neutralisation de la ou des unités structurales de formule (1) est de 50,0 à 100 % en moles, de manière particulièrement préférée de 80,0 à 100 % en moles, de manière notamment préférée de 90,0 à 100 % en moles et de manière exceptionnellement préférée de 95,0 à 100 % en moles.

4. Polymère selon une ou plusieurs des revendications 1 à 3, **caractérisé en ce que** la proportion de la ou des unités structurales de formule (2) dans lesquelles n est un nombre entier supérieur ou égal à 2 dans le composant b) des polymères selon l'invention est d'au moins 10,0 % en moles, de manière particulièrement préférée d'au moins 20,0 % en moles, de manière notamment préférée d'au moins 25,0 % en moles, de manière exceptionnellement préférée d'au moins 30,0 % en moles.

5. Polymère selon une ou plusieurs des revendications 1 à 4, **caractérisé en ce que**, dans la ou les unités structurales de formule (2), B est le groupe -CH₂CH₂- et n est un nombre entier de 1 à 5 et de préférence de 1 à 4.

6. Polymère selon une ou plusieurs des revendications 1 à 5, **caractérisé en ce que** le contre-ion Q⁺ différent de H⁺ dans la ou les unités structurales de formule (1) est choisi parmi NH₄⁺, alcali⁺, alcali⁺ étant à son tour de préférence Na⁺, alcalino-terreux⁺ et les mélanges de ces ions, et est de manière particulièrement préférée NH₄⁺, et le contre-ion X⁺ dans la ou les unités structurales de formule (2) est choisi parmi H⁺, NH₄⁺, alcali⁺, alcali⁺ étant à son tour de préférence Na⁺, alcalino-terreux⁺ et les mélanges de ces ions, et est de manière particulièrement préférée H⁺ et/ou NH₄⁺.

7. Polymère selon une ou plusieurs des revendications 1 à 6, **caractérisé en ce qu'**il contient
a) 50,0 à 96,99 % en moles, de préférence 75,0 à 94,99 % en moles et de manière particulièrement préférée 84,75 à 94,75 % en moles, de la ou des unités structurales de formule (1),
b) 3,0 à 49,99 % en moles, de préférence 5,0 à 24,99 % en moles et de manière particulièrement préférée 5,0 à 15,0 % en moles, de la ou des unités structurales de formule (2), et
c) 0,01 à 5,0 % en moles, de préférence 0,01 à 2,0 % en moles et de manière particulièrement préférée 0,25 à 1,5 % en moles, de la ou des unités structurales réticulantes du composant c).

8. Polymère selon une ou plusieurs des revendications 1 à 7, **caractérisé en ce qu'**il contient une ou plusieurs unités structurales de répétition réticulantes de formule (3) indépendantes les unes des autres 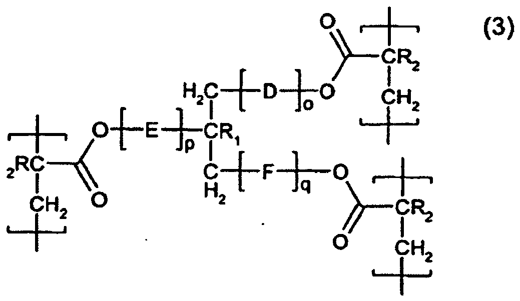 selon le composant c), dans laquelle
R¹ et R² signifient chacun indépendamment l'un de l'autre hydrogène, méthyle ou éthyle,
D, E et F signifient chacun indépendamment les uns des autres méthylèneoxy (-CH₂-O-), éthylèneoxy (-CH₂-CH₂-O-), propylèneoxy (-CH(CH₃)-CH₂-O-), un groupe alkylène linéaire ou ramifié de 1 à 6 atomes de carbone, un groupe alcénylène linéaire ou ramifié, une ou plusieurs fois insaturé, de 2 à 6 atomes de carbone, un groupe monohydroxyalkylène linéaire de 2 à 6 atomes de carbone ou un groupe dihydroxyalkylène linéaire ou ramifié de 3 à 6 atomes de carbone, et
o, p et q signifient chacun indépendamment les uns des autres un nombre entier de 1 à 50.

9. Polymère selon une ou plusieurs des revendications 1 à 8, **caractérisé en ce qu'**il contient
ab) 29,99 à 98,99 % en moles, de préférence 62,0 à 98,99 % en moles, d'un mélange des unités structurales de répétition des formules (1) et (2),
c) 0,01 à 8,0 % en moles de la ou des unités structurales réticulantes du composant c), et
d) 0,01 à 70,0 % en moles, de préférence 1,0 à 70,0 % en moles, de manière particulièrement préférée 1,0 à 37,99 % en moles, d'une ou de plusieurs unités structurales de répétition non réticulantes indépendantes les unes des autres, qui proviennent d'un ou de plusieurs composés de formule générale (4) 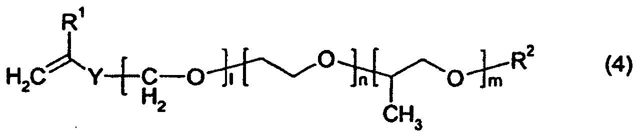 dans laquelle
R¹ signifie hydrogène, méthyle ou éthyle,
R² signifie H, un groupe alkyle linéaire ou ramifié de 1 à 50 atomes de carbone, un groupe monohydroxyalkyle linéaire ou ramifié de 2 à 6 atomes de carbone, un groupe dihydroxyalkyle linéaire ou ramifié de 2 à 6 atomes de carbone, -(CO-O-R⁷-)ₒR⁸ ou - (CO-NR⁵-R⁷-)ₚR⁸,
1, m, n, o et p signifient chacun indépendamment les uns des autres un nombre entier de 0 à 300,
Y signifie une liaison chimique, O, NR³, S, PR³, CH₂, CH₂O, CH₂NR³, CH₂S, C(O), C(NR³), C(O)O, OC(O), C(O)NR³, NR³C(O), C(NR⁴)NR³, C(O)S, R⁶OC(O)O, R⁶OC(O)NR³, R⁶R³NC(O)NR⁴, R⁶NR³C (NR⁵)NR⁴, R⁶OC(O)S, R⁶P(O)O, R⁶OP(O)O, R⁶S(O), R⁶S(O)(O), R⁶S(O)O, R⁶S(O) (O)O, R⁶OS(O)O ou R⁶OS(O) (O)O, de préférence une liaison chimique, O, CH₂, C(O)O, OC(O), C(O)NR³ ou NR³C(O),
R³, R⁴, R⁵ et R⁸ signifient chacun indépendamment les uns des autres l'hydrogène ou un radical alkyle linéaire ou ramifié de 1 à 50 atomes C,
R⁶ signifie une liaison chimique ou CH₂, et
R⁷ signifie un radical alkylène linéaire ou ramifié de 1 à 50 atomes C.

10. Polymère selon la revendication 9, **caractérisé en ce que** la ou les unités structurales du composant d) sont choisies parmi une ou plusieurs unités structurales de formule (5) 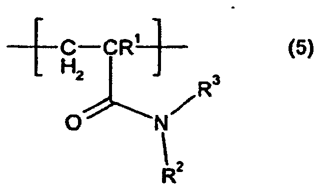 dans laquelle
R¹ signifie hydrogène, méthyle ou éthyle, et
R² et R³ signifient chacun indépendamment l'un de l'autre hydrogène, méthyle, éthyle, n-propyle ou isopropyle, et au moins un des radicaux R² et R³ n'est pas l'hydrogène, et
une ou plusieurs des unités structurales de formule (6) 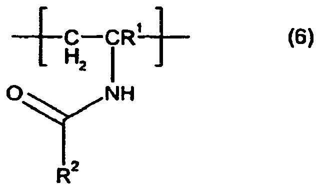 dans laquelle
R¹ signifie hydrogène, méthyle ou éthyle, et
R² signifie hydrogène, méthyle, éthyle, n-propyle ou isopropyle.

11. Procédé de fabrication d'un polymère selon une ou plusieurs des revendications 1 à 10, **caractérisé en ce que** les monomères desquels les unités structurales des composants a) à c) et éventuellement d) dérivent, ainsi qu'éventuellement d'autres monomères, sont polymérisés par voie radicalaire dans un solvant protique, de préférence dans le tert.-butanol, et les monomères avant la polymérisation ou le polymère après la polymérisation sont éventuellement neutralisés avec de l'ammoniac ou des amines organiques ou une base contenant Li⁺, + Na⁺, K⁺, Ca⁺⁺, Mg⁺⁺, Zn⁺⁺ ou Al⁺⁺⁺, de préférence avec les hydroxydes ou carbonates correspondants et de manière particulièrement préférée avec les hydroxydes.

12. Composition cosmétique, dermatologique ou pharmaceutique contenant un ou plusieurs polymères selon une ou plusieurs des revendications 1 à 10, de préférence en une quantité de 0,01 à 10,0 % en poids, de manière particulièrement préférée en une quantité de 0,1 à 5,0 % en poids et de manière notamment préférée en une quantité de 0,5 à 2,0 % en poids, par rapport à la composition finie.

13. Composition selon la revendication 12, **caractérisée en ce qu'**elle se présente sous la forme d'un fluide, d'un gel, d'une huile, d'une mousse, d'un spray, d'une lotion ou d'une crème.

14. Composition selon la revendication 12 ou 13, **caractérisée en ce qu'**elle contient un ou plusieurs tensioactifs, de préférence en une quantité de 0,01 à 50,0 % en poids, de manière particulièrement préférée de 1,0 à 30,0 % en poids, de manière notamment préférée de 5,0 à 20,0 % en poids et de manière exceptionnellement préférée de 5,0 à 15,0 % en poids, par rapport à la composition finie.

15. Composition selon une ou plusieurs des revendications 12 à 14, **caractérisée en ce qu'**elle contient une ou plusieurs substances choisies parmi les sels inorganiques et organiques.

16. Composition selon une ou plusieurs des revendications 12 à 15, **caractérisée en ce qu'**elle contient une ou plusieurs substances choisies parmi les alpha- et bêta-hydroxyacides.

17. Composition selon une ou plusieurs des revendications 12 à 16, **caractérisée en ce qu'**elle contient une ou plusieurs substances choisies parmi la vitamine C et les dérivés de vitamine C, les dérivés de vitamine C étant de préférence choisis parmi l'ascorbylphosphate de sodium, l'ascorbylphosphate de magnésium et l'ascorbylglucoside de magnésium.

18. Composition selon une ou plusieurs des revendications 12 à 17, **caractérisée en ce qu'**elle contient une ou plusieurs substances choisies parmi l'acide benzoïque, l'acide sorbique, l'acide salicylique, l'acide lactique et l'acide paraméthoxybenzoïque.

19. Composition selon une ou plusieurs des revendications 12 à 18, **caractérisée en ce qu'**elle contient une ou plusieurs substances choisies parmi les filtres UV inorganiques et organiques et se présente de préférence sous la forme d'une composition d'écran solaire.

20. Composition selon une ou plusieurs des revendications 12 à 19, **caractérisée en ce qu'**elle présente un pH de 2 à 10, de préférence de 2 à 7 et de manière particulièrement préférée de 2,5 à 6,5.

21. Composition selon une ou plusieurs des revendications 12 à 20, **caractérisée en ce qu'**elle se présente sous la forme d'un gel douche ou d'un shampoing.

22. Utilisation d'un ou de plusieurs des polymères selon une ou plusieurs des revendications 1 à 10 en tant qu'épaississant, donneur de consistance, émulsifiant, additif sensoriel, solubilisant, dispersant, lubrifiant, promoteur d'adhésion, stabilisateur ou créateur de limite d'écoulement, de préférence en tant qu'épaississant, donneur de consistance ou créateur de limite d'écoulement, de manière particulièrement préférée en tant qu'épaississant ou créateur de limite d'écoulement et de manière notamment préférée en tant que créateur de limite d'écoulement, de manière exceptionnellement préférée dans des compositions cosmétiques, dermatologiques ou pharmaceutiques.

23. Utilisation selon la revendication 22 en tant qu'épaississant ou créateur de limite d'écoulement, de préférence en tant que créateur de limite d'écoulement, pour la stabilisation de particules inorganiques ou organiques, de gouttelettes d'huile ou de bulles de gaz, de manière particulièrement préférée dans des compositions cosmétiques, dermatologiques ou pharmaceutiques.

24. Utilisation selon la revendication 22 ou 23 en tant qu'épaississant ou créateur de limite d'écoulement, de préférence en tant que créateur de limite d'écoulement, dans des compositions liquides contenant un ou plusieurs tensioactifs, pour la stabilisation de particules inorganiques ou organiques, de gouttelettes d'huile ou de bulles de gaz, de préférence dans des compositions cosmétiques, dermatologiques ou pharmaceutiques liquides.

25. Utilisation selon la revendication 22 pour la stabilisation d'émulsions, de préférence d'émulsions contenant des sels, de manière particulièrement préférée d'émulsions cosmétiques, dermatologiques ou pharmaceutiques contenant des sels.
